# EUROPÄISCHE PATENTSCHRIFT

(11) **EP 1 503 799 B1**
(45) Veröffentlichungstag und Bekanntmachung des Hinweises auf die Patenterteilung: **30.11.2011**
(21) Anmeldenummer: 03726990.9
(22) Anmeldetag: 15.05.2003
(51) Int. Cl.: A61K 47/48

(54) **IMMUNOGENER, MONOKLONALER ANTIKÖRPER**
IMMUNOGENIC, MONOCLONAL ANTIBODY
ANTICORPS MONOCLONAL IMMUNOGENE

(30) Priorität: 15.05.2002 AT 7442002
(43) Veröffentlichungstag der Anmeldung: 09.02.2005
(73) Patentinhaber: greenovation Biotech GmbH, 79111 Freiburg (DE); Meridian Biopharmaceuticals GmbH, 1230 Wien (AT)
(72) Erfinder: LOIBNER, Hans, A-1238 Wien (AT); WAXENECKER, Günter, A-3240 Mank (AT); HIMMLER, Gottfried, A-1180 Wien (AT); ECKERT, Helmut, CH-4104 Obervil (CH); SCHUSTER, Manfred, A-2191 Schrick (AT); KIRCHEIS, Ralf, A-1230 Wien (AT)
(74) Vertreter: Sonn & Partner Patentanwälte
(86) Internationale Anmeldenummer: PCT/AT2003/000142
(87) Internationale Veröffentlichungsnummer: WO 2003/097663

(56) Entgegenhaltungen:
- WO-A-00/41722
- WO-A-00/67761
- WO-A-01/35989
- WO-A-01/70272
- WO-A-96/22024
- WO-A-02/058728
- BRUMEANU T-D ET AL: "Engineering of doubly antigenized immunoglobulins expressing T and B viral epitopes" IMMUNOTECHNOLOGY, ELSEVIER SCIENCE PUBLISHERS BV, NL, Bd. 2, Nr. 2, 1. Juni 1996 (1996-06-01), Seiten 85-95, XP004052673 ISSN: 1380-2933
- DURRANT L G ET AL: "HUMAN ANTI-IDIOTYPIC ANTIBODIES CAN BE GOOD IMMUNOGENS AS THEY TARGET FC RECEPTORS ON ANTIGEN-PRESENTING CELLS ALLOWING EFFICIENT STIMULATION OF BOTH HELPER AND CYTOTOXIC T-CELL RESPONSES" INTERNATIONAL JOURNAL OF CANCER, NEW YORK, NY, US, Bd. 92, Nr. 3, 1. Mai 2000 (2000-05-01), Seiten 414-420, XP001041422 ISSN: 0020-7136
- MITCHELL M S: "CANCER VACCINES, A CRITCAL REVIEW - PART II" CURRENT OPINION IN INVESTIGATIONAL DRUGS, PHARMAPRESS, US, Bd. 3, Nr. 1, Januar 2002 (2002-01), Seiten 150-158, XP009009457 ISSN: 1472-4472

## Beschreibung

Die vorliegende Erfindung betrifft monoklonale Antikörper, die zur Herstellung von Tumorimpfstoffen geeignet sind, sowie ein Verfahren zur Immunogenisierung von Tumor-assoziierten Antigenen.

Krebszellen haben praktisch immer, neben anderen physiologischen Eigenheiten, die sie von normalen Zellen unterscheiden, eine veränderte Art der Glykosylierung (Glycoconj. J. (1997), 14:569; Adv. Cancer Res. (1989), 52:257; Cancer Res. (1996), 56:5309). Obwohl die Veränderungen von Gewebe zu Gewebe unterschiedlich sind, kann man feststellen, dass eine veränderte Glykosylierung typisch für Krebszellen ist. In den meisten Fällen wird die veränderte Glykosylierung an der Oberfläche der Zellen in Form von Glykoproteinen und Glykolipiden präsentiert. Diese veränderten Zuckerstrukturen können daher als Tumor-assoziierte Antigene (TAA) bezeichnet werden, die in vielen Fällen ausreichend genug tumorspezifisch sind, d.h. sie kommen in "normalen" Zellen selten vor. In vielen Fällen erzeugen die Zellen, und auch die Tumorzellen keine einheitliche Glykosylierung, d.h. es existieren verschiedene Glykoformen von komplexen Glykan-Ketten auf einer Zelle (Annu. Rev. Biochem. (1988), 57:785).

Beispiele für Tumor-assoziierte Kohlenhydratstrukturen sind die Lewis-Antigene, die verstärkt in vielen epithelialen Krebsarten exprimiert werden. Dazu gehören Lewis x-, Lewis b- und Lewis y-Strukturen, sowie sialylierte Lewis x-Strukturen. Andere Kohlenhydrat-Antigene, sind GloboH-Strukturen, KH1, Tn-Antigen, TF-Antigen, das alpha-1,3-Galactosyl-Epitop (Elektrophoresis (1999), 20:362; Curr. Pharmaceutical Design (2000), 6:485, Neoplasma (1996), 43:285).

Andere TAA sind Proteine, die auf Krebszellen besonders stark exprimiert werden, wie z.B. CEA, TAG-72, MUC1, Folate Binding Protein A-33, CA125,EpCAM, HER-2/neu, PSA, MART, etc. (Sem. Cancer Biol. (1995), 6:321).

Ein Ansatz, Tumorzellen relativ spezifisch zu zerstören, ist die passive Immuntherapie mit Antikörpern, die gegen TAA gerichtet sind (Immunology Today (2000), 21:403-410; Curr. Opin. Immunol. (1997), 9:717).

Ein anderer Ansatz, Tumorzellen zu zerstören, ist eine aktive Impfung, die eine Immunantwort gegen TAA auslöst. Diese Immunantwort ist somit auch gegen die entsprechenden Tumorzellen gerichtet (Ann. Med. (1999), 31:66; Immunobiol. (1999), 201:1).

Im Fall einer aktiven Immunisierung ist die schwache Immunogenität der Antigene ein großes Problem. Kohlenhydrate sind sehr kleine Moleküle und werden daher vom Immunsystem nicht direkt erkannt. Kohlenhydrate und Polysaccharide werden allgemein als Thymus-unabhängige Antigene betrachtet. Konjugation von immunologisch inerten Kohlenhydratstrukturen mit Thymus-abhängigen Antigenen, wie Proteinen, verstärken ihre Immunogenität. Impfstoffe, die auf tumor-assoziierten Kohlenhydrat-Strukturen basieren, werden daher an sogenannte "Trägermoleküle" gekoppelt, um die Immunogenität zu erhöhen (Angew. Chem. Int. Ed. (2000), 39:836). Als Trägermoleküle dienen oft Proteine, wie bovines Serumalbumin oder KLH (Keyhole Limpet Hemocyanin). Das Protein stimuliert dabei die Träger-spezifischen T-Helfer-Zellen, die dann eine Rolle bei der Induktion der anti-Kohlenhydrat-Antikörper-Synthese spielen (Contrib. Microbiol. Immunol. (1989), 10:18).

Darüber hinaus sind sowohl die Kohlenhydrat-Antigene als auch die Protein-Antigene auch auf gesunden Geweben (zumindest in bestimmten Entwicklungsstufen des Organismus) vorhanden und werden deswegen vom Immunsystem als autologes Material erkannt - dadurch gibt es in der Regel keine Immunantwort gegen diese körpereigenen Moleküle.

Eine weitere Möglichkeit, auch die Qualität der Immunantwort auf Kohlenhydrate zu verbessern, ist die Immunisierung mit sogenannten "Mimotopen.", die keine Kohlenhydrate sind (z.B. Peptide; Nat. Biotechnol. (1999), 17:660; Nat. Biotechnol. (1997), 15:512).

Tumor-assoziierte Proteine sind schwach immunogen, werden aber trotzdem als Impfstoffe in Betracht gezogen (Ann. Med. (1999), 31:66; Cancer Immunol. Immunother. (2000), 49:123; US-Patent 5994523). Eine Möglichkeit, die "Selbst"-Erkennung von bestimmten TAA zu umgehen, ist die Verwendung von antiidiotypischen Antikörpern als Immunogen, die die Struktur des TAA nachahmen und so eine Immunantwort auslösen, die auch mit dem TAA reagiert (Cancer Immunol.Immunother. (2000), 49:133). Es gibt noch andere Möglichkeiten, die Selbst-Toleranz gegenüber bestimmten Proteinen zu durchbrechen, wie z.B. die Fusion des TAA mit bestimmten fremden Proteinsequenzen (US 5,869,057, US 5,843,648 und US 6,069,242) oder über entsprechende Präsentation durch Antigenpräsentierende Zellen (Immunobiol. (1999), 201:1).

Aufgabe der vorliegenden Erfindung ist die Vermeidung der Nachteile der im Stand der Technik beschriebenen Tumorvakzine und entsprechend eine verbesserte Tumorvakzine zur Verfügung zu stellen, die eine effiziente Immunantwort gegen Tumorzellen hervorruft.

Diese Aufgabe wird erfindungsgemäß durch einen immunogenen Antikörper gelöst, der in dem Anspüchen definiert ist.

Unter dem Begriff "immunogen" sind alle Strukturen zu verstehen, die zu einer Immunantwort in einem spezifischen Wirtssystem führen. Beispielsweise wirkt ein muriner Antikörper oder Fragmente dieses Antikörpers sehr stark immunogen im menschlichen Organismus, eine Wirkung, die durch die Kombination mit Adjuvantien noch verstärkt werden kann.

Ein immunogener Antikörper kann durch seine Spezifität oder seine Struktur immunogen wirken. Bevorzugt kann der erfindungsgemäße immunogene Antikörper auch im denaturierten Zustand oder als Konjugat mit ausgewählten Strukturen oder Trägerstoffen Immunogenität induzieren.

Der Begriff "Epitop" definiert jede Region innerhalb eines Moleküls, die durch einen spezifischen Antikörper erkannt werden kann oder die die Bildung dieser spezifischen Antiköper induziert. Epitope können Konformations-Epitope oder lineare Epitope sein.

Die Epitope imitieren oder umfassen vor allem Domänen eines natürlichen, homologen oder derivatisierten TAA. Diese sind zumindest durch ihre Primärstruktur und eventuell Sekundärstruktur mit den TAA vergleichbar. Die Epitope können sich aber auch von den TAA darin völlig unterscheiden, und rein durch die Ähnlichkeit von räumlichen (Tertiär-) Strukturen Bestandteile eines TAA, vor allem proteinärer bzw. Kohlenhydrat-Antigene nachahmen. Allein die Tertiärstruktur eines Moleküls kann somit ein Mimik ("immunological imitation"; wie z.B. in der WO 00/73430 offenbart) bilden, das die Immunantwort gegen ein bestimmtes TAA hervorruft.

In der Regel ist davon auszugehen, dass unter einem Antigen, das ein proteinäres Epitop eines Tumor-assoziierten Antigens imitiert, ein Polypeptid von mindestens fünf Aminosäuren zu verstehen ist.

Unter den Epitopen des erfindungsgemäßen Antikörpers findet sich vorzugsweise mindestens ein Epitop eines Antigens ausgesucht aus der Gruppe der Peptide oder Proteine, insbesondere EpCAM, NCAM, CEA und T-Zell Peptide, welche vorzugsweise von Tumor-assoziierten Antigenen abgeleitet sind, weiter der Kohlenhydrate, insbesondere Lewis Y, SialylTn, Globo H, und der Glycolipide, insbesondere GD2, GD3 und GM2. Bevorzugte Epitope sind von Antigenen abgeleitet, die spezifisch für epitheliale Tumoren sind und etwa vermehrt bei Brustkrebs, Krebs des Magen und Darms, der Prostata, Pankreas, Ovarien und der Lunge vorkommen. Unter den bevorzugten Epitopen sind diejenigen zu finden, welche vor allem eine humorale Immunantwort, also eine spezifische Antikörperbildung *in vivo* hervorrufen. Der erfindungsgemäße immunogene Antikörper kann vorzugsweise auch eine T-Zell spezifische Immunantwort auslösen, wodurch als Reaktion auf die Verabreichung des Antikörpers nicht nur Antikörper beispielsweise der IgM-Klasse gebildet werden, sondern auch der IgG-Klasse.

Alternativ können speziell auch diejenigen Antigene als Epitope im Sinne der Erfindung ausgewählt werden, welche eine T-Zellspezifische Immunantwort generieren. Darunter sind vor allem auch intrazelluläre Strukturen bzw. T-Zell Peptide zu finden.

Weiter bevorzugte proteinäre Epitope, die besonders auf Krebszellen solider Tumoren exprimiert werden, sind z.B. TAG-72, MUC1, Folate Binding Protein A-33, CA125, HER-2/neu, EGF-Rezeptoren, PSA, MART etc. (s. z.B. Sem. Cancer Biol. 6 (1995), 321). Weiters können auch sogenannte T-Zell-Epitop-Peptide (Cancer Metastasis Rev. 18 (1999), 143; Curr. Opin. Biotechnol. 8 (1997), 442; Curr. Opin. Immunol. 8 (1996), 651) oder Mimotope solcher T-Zell-Epitope (Curr. Opin. Immunol. 11 (1999), 219; Nat. Biotechnol. 16 (1998), 276-280) dienen. Geeignete Epitope werden zumindest in 20%, vorzugsweise mindestens in 30% der Fälle von Tumorzellen einer bestimmten Krebsart exprimiert, weiter bevorzugt in zumindest 40%, insbesondere in zumindest 50% der Patienten.

Erfindungsgemäß bevorzugte Kohlenhydrat-Epitope sind Tumor-assoziierte Kohlenhydratstrukturen, wie die Lewis-Antigene, z.B. Lewis x-, Lewis b- und Lewis y-Strukturen, sowie sialylierte Lewis x-Strukturen. Weiters sind auch GloboH-Strukturen, KH1, Tn-Antigen, besonders bevorzugt SialylTn-Antigen, TF-Antigen, das alpha-1-3,Galactosyl-Epitop bevorzugte Kohlenhydrat-Antigen-Strukturen im Rahmen der vorliegenden Erfindung.

Am Antikörper Können auch zumindest zwei gleiche oder verschiedene Epitope eines Adhäsionsproteins, etwa eines homophilen zellulären Membranproteins, wie EpCAM, vorgesehen bzw. imitiert werden. Somit kann durch die aktive Immunisierung eine Vielzahl von Antikörpern mit Spezifität für dasselbe Molekül, aber unterschiedlichen EpCAM-Bindungsstellen generiert werden.

Der erfindungsgemäße Antikörper ist ein glykosilierter Antikörper, wobei die Glykosilierung selbst auch ein Epitop eines Kohlenhydrat-Epitopes eines TAA imitieren kann.

Es sind mindestens zwei verschiedene Epitope vorgesehen bzw. imitiert, wobei mindestens ein Epitop aus der Gruppe der Peptide oder Proteine und mindestens ein Epitop aus der Gruppe der Kohlehydrate stammt. Es hat sich herausgestellt, dass ein Epitop eines EpCAM-Proteins und ein Epitop eines Kohlenhydratbestandteils, etwa von Lewis Y, oder SialylTn, bevorzugt kombiniert werden. Insbesondere stellt ein Lewis Y-glycosilierter Antikörper mit einer Spezifität für eine EpCAM-Struktur ein besonders gutes Immunogen in einer Impfstoff-Formulierung dar. Dieser Antikörper kann besonders gut zelluläre Tumorantigene nachahmen, und bewirkt dementsprechend die gewünschte Immunantwort zur Inhibition von epithelialen Tumorzellen.

Bevorzugt wirkt der erfindungsgemäße immunogene Antikörper als Antigen-Träger, beispielsweise eines proteinären Antigens, im Impfstoff. Das heißt, der erfindungsgemäße Antikörper stellt ein multivalentes Antigen dar, beispielsweise ein bi-, tri- oder polyvalentes Antigen. Die Epitope werden so präsentiert, dass der Impfstoff eine Immunantwort gegen diese Epitope hervorruft. Damit wird ein Impfstoff zur Verfügung gestellt, der einen Antikörper als ein di-, tri- oder polyvalentes Antigen enthält.

Der erfindungsgemäße Antikörper wird vorwiegend zur aktiven Immunisierung eingesetzt, und daher nur in geringen Mengen verabreicht. So werden etwa keine besonderen Nebenwirkungen erwartet, auch wenn der erfindungsgemäße Antikörper von einer nicht-humanen Spezies abgeleitet ist, wie etwa ein muriner Antikörper. Es wird jedoch angenommen, dass ein rekombinanter, chimärer, sowie ein mit murinen und menschlichen Bestandteilen kombinierter, humanisierter oder humaner Antikörper für die Verabreichung am Menschen besonders verträglich ist. Andererseits kann ein muriner Anteil im erfindungsgemäßen Antikörper durch seine Fremdartigkeit die Immunantwort im Menschen noch zusätzlich provozieren.

Bevorzugte primäre Funktion des erfindungsgemäßen immunogenen Antikörpers ist das Präsentieren der Epitope. Die spezifische Erkennung des Tumor-assoziierten Antigens bzw. der Tumor-assoziierten Antigene, deren Epitope er aufweist, ist nicht unbedingt notwendig, er kann aber zusätzlich spezifisch an ein Epitop binden und gleichzeitig ein Epitop präsentieren.

Obwohl natürlich ein erfindungsgemäßer Antikörper von einem nativen Antikörper abgeleitet sein kann, der eventuell aus einem Organismus oder Patienten isoliert wurde, wird üblicherweise ein Antikörper-Derivat eingesetzt, das bevorzugt aus der Gruppe der Antikörper-Fragmente, -Konjugate oder -Homologe, aber auch Komplexe und Adsorbate ausgesucht ist. Jedenfalls ist es bevorzugt, dass das Antikörper-Derivat zumindest Teilen des Fab-Fragmentes enthält, bevorzugt gemeinsam mit zumindest Teilen des F(ab')₂ Fragmentes, und/oder Teilen der hinge Region und/oder des Fc-Teils eines lambda oder kappa Antikörpers.

Weiter kann auch ein einkettiges Antikörper-Derivat, etwa ein sogenannter "single chain" Antikörper als Träger der Epitope im Sinne der Erfindung herangezogen werden. Der erfindungsgemäße Antikörper ist vorzugsweise von der Art eines Immunglobulins, etwa eines IgG, IgM oder IgA.

Am erfindungsgemäßen Antikörper können zusätzlich noch andere Stoffe kovalent in der Molekülstruktur enthalten sein, wie Peptide, Glycopeptide, Kohlenhydrate, Lipide oder Nukleinsäuren, aber auch ionische Gruppen, wie Phosphatgruppen, oder auch Trägermoleküle, wie Polyethylenglycol oder KLH. Diese Seitengruppen können unter Umständen selbst Epitope eines Tumor-assoziierten Antigens im Sinne der vorliegenden Erfindung repräsentieren.

In der erfindungsgemäßen Tumorvakzine werden insbesondere anti-idiotypische Antikörper, also ab2, zur aktiven Immunisierung eingesetzt. Diese Antikörper können mit zusätzlichen Sequenzen bzw. Strukturen ausgestattet werden, um ein erfindungsgemäßes Immunogen zu erhalten. Erfindungsgemäße anti-idiotypische Antikörper erkennen bevorzugt wieder den Idiotyp eines Antikörpers, der gegen ein TAA gerichtet ist. Damit wird schon ein Epitop eines TAA am Paratop des anti-idiotypischen Antikörpers als Mimik für das TAA ausgebildet. Auch hier wird die Auswahl der Epitope bevorzugt aus den oben erwähnten Gruppen der TAA vorgenommen. Beispielhaft wird ein anti-idiotypischer Antikörper gegen Glykan-spezifische Antikörper eingesetzt, etwa ein anti-idiotypischer Antikörper, der den Idiotyp eines anti-Lewis Y Antikörpers erkennt, z.B. wie in der EP 0 644 947 beschrieben.

Der erfindungsgemäße immunogene Antikörper eignet sich vor allem als Basis für pharmazeutische Präparationen, insbesondere von Impfstoffen. Bevorzugt sind pharmazeutische Präparationen, welche einen pharmazeutisch akzeptablen Träger enthalten. Dieser umfasst beispielsweise Hilfsstoffe, Puffer, Salze, Konservierungsmittel. Die pharmazeutischen Präparationen können z. B. zur Prophylaxe und Therapie von Krebs-assoziierten Krankheitszuständen, wie der Metastasenbildung von Krebspatienten eingesetzt werden. Dabei werden *in vivo* oder auch *ex vivo* Antigen-präsentierende Zellen spezifisch moduliert, um die Immunantwort gegen die TAA zu generieren, die der immunogene Antikörper aufweist.

Eine erfindungsgemäß bevorzugte Impfstoff-Formulierung enthält den immunogenen Antikörper zumeist nur in geringen Konzentrationen, etwa in einer immunogenen Menge im Bereich von 0.01 µg bis 10 mg. Je nach Natur des Antikörpers, sei es durch speziesfremde Sequenzen oder Derivatisierung, aber auch je nach verwendeten Hilfsmitteln bzw. Adjuvantien, wird die geeignete immunogene Dosis gewählt, etwa im Bereich von 0.01 µg bis 750 µg, vorzugsweise 100 µg bis 500 µg. Ein Depot-Impfstoff, der an den Organismus über einen längeren Zeitraum abgegeben werden soll, kann aber auch weit höhere Antikörpermengen enthalten, etwa mindestens 1 mg bis zu mehr als 10 mg. Die Konzentration richtet sich nach der verabreichten Menge des flüssigen oder suspendierten Impfstoffes. Ein Impfstoff wird üblicherweise in Fertigspritzen mit einem Volumen von 0.01 bis 1 ml, vorzugsweise 0.1 bis 0.75 ml, zur Verfügung gestellt. Es handelt sich also um konzentrierte Lösungen bzw. Suspensionen.

Der immunogene Antikörper wird in dem erfindungsgemäßen Impfstoff vorzugsweise in einem pharmazeutisch akzeptablen Träger präsentiert, der sich für die subkutane, intramuskuläre aber auch intradermale oder transdermale Administration eignet. Eine weitere Art der Verabreichung funktioniert über den mucosalen Weg, etwa die Vakzinierung durch nasale oder perorale Verabreichung. Wenn Feststoffe als Hilfsmittel für die Impfstoff-Formulierung herangezogen werden, wird etwa ein Adsorbat bzw. eine suspendierte Mischung des Antikörpers mit den Hilfsmittel verabreicht. In besonderen Ausführungsformen wird der Impfstoff als Lösung bzw. Flüssigimpfstoff in einem wässerigen Lösungsmittel dargereicht.

Vorzugsweise werden Impfeinheiten der Tumorvakzine bereits in einer geeigneten Fertigspritze zur Verfügung gestellt. Nachdem ein Antikörper im Vergleich zu den TAA relativ stabil ist, hat der erfindungsgemäße Impfstoff den wesentlichen Vorteil, dass er bereits als lagerstabile Lösung bzw. Suspension in einer gebrauchsfertigen Form "ready-to-use" in den Verkehr gebracht werden kann. Ein Gehalt an Konservierungsmittel, wie Thimerosal oder andere Konservierungsmittel mit verbesserter Verträglichkeit, ist zwar nicht notwendigerweise erforderlich, kann aber zur längeren Haltbarkeit bei Lagertemperaturen von Kühlschranktemperaturen bis zu Raumtemperatur in der Formulierung vorgesehen werden. Der erfindungsgemäße Impfstoff kann aber auch in gefrorener oder lyophilisierter Form zur Verfügung gestellt werden, der bei Bedarf aufgetaut bzw. rekonstituiert werden kann.

Jedenfalls hat es sich bewährt, die Immunogenität des erfindungsgemäßen Antikörpers durch die Verwendung von Adjuvantien zu erhöhten. Dafür sind Vakzine-Adjuvantien der beispielsweise Aluminiumhydroxid (Alu-Gel) oder -phosphat, z.B. Wachstumsfaktoren, Lymphokine, Zytokine, etwa IL-2, IL-12, GM-CSF, Gamma Interferon, oder Komplementfaktoren, wie C3d, weiter Liposomenbereitungen oder Lipopolysaccharid aus E. coli (LPS) aber auch Formulierungen mit zusätzlichen Antigenen, gegen die das Immunsystem bereits eine starke Immunantwort gemacht hat, wie Tetanus-Toxoid, Bakterielle Toxine, wie Pseudomonas-Exotoxine und Derivate von Lipid A.

Zur Impfstoff-Formulierung können auch weiter bekannte Verfahren zur Konjugierung oder Denaturierung von Impfstoff-Bestandteilen eingesetzt werden, um die Immunogenität des Wirkstoffes noch zu erhöhen.

Besondere Ausführungsformen des erfindungsgemäßen Impfstoffes enthalten weitere Impfantigene, insbesondere anti-idiotypische Antikörper, also Mischungen des erfindungsmäßen immunogenen Antikörpers mit verschiedenen Antikörpern, die gleichzeitig verabreicht werden.

Der erfindungsgemäße immunogene Antikörper eignet sich auch zur Herstellung von erfindungsgemäßen diagnostischen Mitteln. So können etwa Reagenzien enthaltend den immunogenen Antikörper gemeinsam mit weiteren Reaktanden bzw. Detektionsmitteln als diagnostisches Mittel in Form eines Sets angeboten werden. Ein derartiges Mittel enthält bevorzugterweise eine Markierung ("label") zur unmittelbaren Detektion des Antikörpers bzw. dessen Reaktionsproduktes. Das erfindungsgemäße diagnostische Mittel wird beispielsweise zur qualitativen und/ oder quantitativen Bestimmung von Tumorzellen bzw. Metastasen oder zur Bestimmung eines metastasenbildenden Potentials eingesetzt, wobei das Mittel durch eine Immunreaktion oder Immunkomplexbildung wirkt.

Das erfindungsgemäße Verfahren umfasst folgende Verfahrenschritte :
a)Bereitstellen eines Antikörpers mit dem Idiotyp eines Tumor-assoziierten Antigens und
b)Kopplung von mindestens einem Epitop eines Tumor-assoziierten Antigens an den Antikörper.

Die Kopplung wird üblicherweise durch chemische oder biologische, etwa enzymatische, Reaktion vorgenommen. Eine Verbindung eines Antikörpers mit einem Epitop kann aber auch bereits auf molekularbiologischer Ebene erfolgen. Durch Rekombination von Nukleinsäuren kann bereits ein konjugiertes Produkt exprimiert und hergestellt werden. Derartige erfindungsgemäße Verfahren sind durch die Schritte
a)Bereitstellen einer Nukleinsäure kodierend für einen Antikörper mit dem Idiotyp eines Tumor-assoziierten Antigens und
b)Rekombinieren der Nukleinsäure mit einer Nukleinsäure, die für ein Epitop eines Tumor-assoziierten Antigens oder dessen Mimik kodiert.
gekennzeichnet.

Der Antikörper, von dem erfindungsgemäß ausgegangen wird, ist ein anti-idiotypischer Antikörper, also ein ab2.

Die Kopplung entspricht einer Konjugierung zu Ausbildung einer kovalenten Bindung. Dadurch wird ein Derivat synthetisiert, das sich von nativen Antikörpern unterscheidet.

Die erfindungsgemäße Kombination von zwei in ihrer Natur völlig verschiedenen immunogenen TAA-Mimiks ermöglicht überraschenderweise eine äußerst effiziente Immunisierung gegen Tumor-assoziierte bzw. Tumor-spezifische Strukturen, so dass das körpereigene Immunsystem effizient gegen die jeweiligen Tumore geschützt werden kann bzw. gegen diese Tumore ankämpfen kann.

Der erfindungsgemäße Antikörper fungiert als proteinärer Antigen-Träger, der beispielsweise mit einem Kohlenhydrat-Antigen als Konjugat der vorliegenden Erfindung vorliegt. Gleichfalls ist es auch möglich, mehrere Kohlenhydrat-Antigene im erfindungsgemäßen Konjugat vorzusehen. So können beispielsweise auf einem Antikörper mehrere verschiedene Glykane gekoppelt sein, die Immunantworten gegen zwei oder mehrere verschiedene Tumor-assoziierte Kohlenhydrat-Strukturen auslösen. Ein solches Konjugat kommt in natürlichen Systemen nicht vor. Die autoantigenen Strukturen werden dadurch als fremdartig erkannt, was die Immunogenität zusätzlich verstärkt. Erfindungsgemäß liegt ein solches Konjugat daher in einer synthetischen Anordnung vor, die weder sterisch noch funktionell natürlicherweise (d.h. in Tumorzellen) vorkommt.

Die erfindungsgemäße Kopplung von zwei von ihrer Natur her völlig unterschiedlichen Strukturen auf einem Molekül hat neben dem Vorteil einer einfachen Formulierung des synthetischen Impfstoffes auch ein sehr viel einfacheres Impfschema zur Folge, da immer mit dem gleichen Impfstoff gearbeitet werden kann: Sowohl die initiale Impfung, als auch spätere Boosterimpfungen werden vorzugsweise mit dem gleichen Impfstoff vorgenommen.

Offenbart ist auch ein Verfahren zur Immunogenisierung von Epitopen von Tumor-assoziierten Antigenen oder deren Mimik. Dafür werden vor allem niedermolekulare Epitope der Antigene herangezogen, die für sich alleine vom Immunsystem der Säugetiere, insbesondere des Menschen, kaum erkannt werden. Die Immunogenisierung erfolgt derart, dass ein Antigen an einem Antikörper konjugiert wird, wobei der Antikörper als Träger fungiert. Mit dem Verfahren kann eine Vielzahl von Epitopen immunogen gemacht werden, insbesondere natürlich die Epitope der bereits genannten Auswahl von Antigenen. Der hergestellte immunogene Antikörper enthält vorzugsweise das zu immunogenisierende Epitop und ein weiteres Epitop eines Tumor-assoziierten Antigens.

Durch die Immunogenisierung wird ein Material erhalten, das sich überraschend gut zur Immunisierung von Patienten eignet. Das erfindungsgemäß erhältliche Produkt wird daher vorzugsweise als Impfstoff zur Verfügung gestellt.

Verfahren zur Auffindung geeigneter antigener Strukturen, Modellierung und Herstellung der von TAA abgeleiteten Peptide, Polypeptide oder Proteine bzw. dafür kodierende Nukleinsäuren, weiter Lipoproteine, Glycolipide, Kohlenhydrate oder Lipide sind dem Fachmann bekannt und können ohne unzumutbaren experimentellen Aufwand für die jeweilige Tumor-spezifische Struktur zur Verfügung gestellt werden. Weiter sind die Verfahren zur Konjugierung eines Proteins mit derartigen Strukturen bekannt, welche für das erfindungsgemäße Verfahren geeignet sind.

Die als Epitop-Mimik gewählten Kohlenhydratstrukturen können aus natürlichen oder synthetischen Quellen stammen, wobei die Kohlenhydrate als Glykoproteine bzw. als Glykolipide vorliegen und als solche an das entsprechende Trägermolekül gekoppelt werden können.

Auch die Antikörper-Bestandteile können chemisch synthetisiert und anschließend mit Epitop-Strukturen verbunden werden bzw. gemeinsam synthetisiert werden. Bei einer chemischen Synthese von Antikörper-Trägermolekülen ist es möglich, an besonderen Stellen reaktive Gruppen einzuführen, um sowohl das Ausmaß der Kopplung mit einem Epitop als auch die Art und den Ort der Bindung kontrollieren zu können.

Die Antikörper-Träger können auch gentechnisch als rekombinante Moleküle hergestellt werden. Es ist denkbar, diese Antikörper in Wirtszellen zu produzieren, die keine Glykosylierung vornehmen (wie z.B. Escherichia coli). Derartige Polypeptide können dann chemisch oder enzymatisch mit einem gewünschten Kohlenhydrat-Antigen gekoppelt werden.

Es ist aber auch denkbar, dass der Antikörper-Träger in Zellen produziert wird, die das Molekül glykosylieren können. Die gentechnische Veränderung von Nukleinsäuren, die für native Antikörper kodieren, kann etwa bewirken, dass im translatierten Molekül entsprechende Glykosylierungsstellen entstehen.

Eine Glykosylierung eines derartigen rekombinanten Genproduktes mit den entsprechenden Tumor-assoziierten Glykanstrukturen kann durch Produktion in Zellen erfolgen, die genetisch so verändert sind, dass sie Proteine entsprechend glykosylieren. Solche Zellen können natürliche Isolate (Zellklone) sein, die durch entsprechendes Screening auf die gewünschte Glykosylierung gefunden werden können.

Es können aber auch Zellen derart modifiziert werden, dass sie entsprechende Enzyme, die für die gewünschte Glykosylierung notwendig sind, so exprimieren, dass eben die gewünschte Glykosylierung an dem rekombinanten Polypeptid-Träger-Protein zu finden ist (Glycoconj. J. (1999), 16: 81).

Es ist aber auch möglich, das Glykosylierungsmuster von Proteinen enzymatisch herzustellen, bzw. zu verändern (Clin. Chem. Lab. Med. (1998), 36: 373).

Im erfindungsgemäßen immunogenen Antikörper können die verschiedenen Epitop-Strukturen über einen Koppler miteinander verbunden sein. Dieser Koppler ist vorzugsweise ein kurzes bifunktionelles Molekül, wie z.B. N-Hydroxysuccinimid. Die Kopplung über Nitrophenyl-aktivierte Zucker ist ebenfalls möglich. In einer bevorzugten Ausführungsform erfolgt die Kopplung über Sulfhydryl-Gruppen (Biochim.Biophys.Acta (1983), 761, 152-162). Beispiele für Sulfhydryl-reaktive Linker sind BMH, DFDNB oder DPDPB. Gleichwohl kann der Koppler auch durch eine größere chemische Verbindung als ein einfaches Kopplermolekül verwirklicht werden. Voraussetzung ist immer, dass dieser Koppler keinen negativen Einfluss auf die immunologischen Eigenschaften des Konjugates hat, d.h., dass er selbst keine wesentliche Immunogenität auslöst. Ein Koppler kann erfindungsgemäß auch durch die chemische Umwandlung eines Teiles des Antikörpers bzw. der zu konjugierenden Struktur quasi "in situ" hergestellt werden. Dieser am Antikörper bzw. an der Epitopstruktur selbst hergestellte Koppler kann dann direkt zum jeweils anderen Bindungspartner konjugiert werden (z.B. über die Amingruppe des Lysins, über OH-Gruppen, Schwefelgruppen, etc.). Kopplungsverfahren sind aus dem Stand der Technik bekannt (Anal.Biochem, (1986) 156, 220-222; Proc. Natl.Acad.Sci., (1981), 78, 2086-2089; Biochem.Biophys.Res. Comm. (1983), 115, 29-37).

Gemäß einer besonderen Ausführungsform der vorliegenden Erfindung umfasst der erfindungsgemäße Antikörper ein Nukleinsäure-Molekül, das für ein proteinäres TAA als Epitop-Struktur im Sinne der vorliegenden Erfindung kodiert, wobei die Nukleinsäure kovalent konjugiert ist.

Offenbart ist auch ein Set, das geeignet zur Tumorvakzinierung ist. Das Set umfasst eine Präparation eines erfindungsgemäßen immunogenen Antikörpers und eine geeignete Applikationsvorrichtung, wie z.B. Spritzen, Infusionsvorrichtung, etc. Wenn die Konjugatpräparation in lyophilisierter Form vorliegt, enthält das Set weiter eine geeignete Rekonstituierungslösung, die gegebenenfalls spezielle Stabilisatoren oder Rekonstituierungsbeschleuniger aufweist.

Mit der vorliegenden Erfindung, bei welcher der immunogene Antikörper mit mehreren verschiedenen Epitop-Strukturen, insbesondere mit der Struktur eines Tumor-assoziierten Kohlenhydrat-Antigens, zur Verfügung gestellt werden, ist es möglich, eine Immunantwort auszulösen, die zwei oder mehrere Spezifitäten besitzt und dadurch eine Tumorzelle mit zwei oder mehreren verschiedenen Tumor-assoziierten Antigenen bekämpft. Dadurch wird der Wirkungsbereich der Vakzine verbreitert und spezifischer gestaltet.

Die Erfindung wird durch die nachfolgenden Beispiele sowie durch die Zeichnungsfiguren näher erläutert, ohne jedoch darauf beschränkt zu sein.

Es zeigen:
Fig. 1 die Erkennung der Bispezifität des Neoglykoproteins HE2-LeY durch spezifische Antikörper;
Fig. 2 einen Sandwich-ELISA mit beschichtetem anti-LeY-Antikörper und Detektion mit anti-HE2-Antikörper;
Fig. 3 SDS-PAGE verschiedener Neoglykokonjugate;
Fig. 4 Immuno-Western Blot von SDS-PAGE verschiedener Neoglykokonjugat;
Fig. 5 HE2-ELISA; und
Fig. 6 LeY-PAA-ELISA.
Fig. 7 das LDS PAGE. Ein Vergleich zwischen HE2 (lanes 2-5) und demm HE2-SialylTn Kopplungsprodukt (lanes 6-8) zeigt einen klaren Anstieg im Molekulargewicht der schweren Kette. Dies bedeutet, dass SialylTn erfolgreich an die schwere Kette (50 kDa) des HE2 Antikörpers gekoppelt wurde. Zusätzlich zeigt das Auftreten einer zweiten Bande (mit geringfügig höherem Molekulargewicht) zur 25 kDa Bande, dass auch SialylTn auch teilweise an die leichte Kette gekoppelt wurde.
Fig. 8 den Antikörper Titer gegen HE2. Die Induktion der Immunantwort gegen HE2 durch die HE2-SialylTn Multiepitop-Vakzine ist vergleichbar mit jener, die durch HE2 hervorgerufen wird.
Fig. 9 den SialylTn-PAA ELISA
Fig. 10 die EpCAM Affinitätschromatographie. Die Ergebnisse zeigen, dass die Bindung der durch die HE2-SialylTn Vakzine induzierten Antikörper gegen EpCAM im Serum nach der Immunisierung vergleichbar ist mit jener von HE2.

### Beispiele:

### Beispiel 1: Kopplung eines Lewis Y-Kohlenhydrates an einen EpCAM-spezifischen Antikörper

Der Antikörper HE2 ist in der Patentanmeldung WO 00/41722 beschrieben und hat die Eigenschaft, bei entsprechender Immunisierung eine Immunantwort, die an Tumorzellen bindet, hervorzurufen. Ein synthetisch hergestelltes Lewis Y-Kohlenhydrat-Antigen wird erfindungsgemäß an HE2 gekoppelt. Im vorliegenden Beispiel erfolgt die Kopplung chemisch:
Der Antikörper HE2 wird in einem geeignetem Puffer (100 mM Natrium-Phosphatpuffer mit 150 mM NaCl, pH 8,5) mit N-Hydroxysuccinimid aktiviertem synthetischen Lewis Y-Tetrasaccharid (Syntesome GmbH, München, Deutschland) gekoppelt.

N-Hydroxysuccinimid-aktiviertes Lewis Y-Tetrasaccharid wird in N,N-Dimethylformamid gelöst (100 mg/ml) und tropfenweise zur HE2- Antikörperlösung in entsprechendem Puffer (100 mM Natrium-Phosphatpuffer mit 150 mM NaCl, pH 8,5) zugesetzt und bei 4°C mindestens 2,5 Stunden geschüttelt. Das Ausmaß der Glykosylierung des Antikörpers mit Lewis Y lässt sich durch die Wahl des molaren Überschusses an aktiviertem Zucker sowie der Konzentration der antikörperhältigen Lösung (1-10 mg/ml) steuern. Für vergleichszwecke werden zwei verschiedene Reaktionsansätze durch Variation des molaren Überschusses (5fach bzw. 15fach) an aktiviertem Zucker hergestellt: "Neoglykoprotein I" mit niedrigerem Kohlenhydratanteil und "Neoglykoprotein II" mit höherem Kohlenhydratanteil.

Die Bispezifität des Neoglykoproteins lässt sich mit verschiedenen immunologischen Methoden (ELISA oder Western Blotting mit Antikörpern, die gegen die Lewis Y-Determinante oder gegen HE2 gerichtet sind) nachweisen.

### Direkter ELISA:

HE2, HE2-Lewis Y-Neoglykoprotein oder LeY-PM (Polyacrylamidgekoppeltes Tetrasaccharid, Syntesome 045-PA) wird in Beschichtungs-Puffer (15 mM Na₂CO₃, 5 mM NaHCO₃, 3mM NaN₃, pH 9,6) gelöst (10 µg/ml) und auf eine Mikrotiterplatte (Nunc, Dänemark, Maxisorb) gebunden (1 Stunde bei 37°C, 100 µl/well). Nach dreimaligem Waschen der Mikrotiterplatten mit Waschpuffer (2% NaCl, 0,2% Triton X-100 in PBS; 200 µl) wird mit 5% fötalem Kälberserum in PBS (138 mM NaCl, 1,5 mM KOH, 2,7 mM KCl, 6,5 mM Na₂HPO₄ pH 7,2; 200 µl) blockiert (30 Minuten bei 37°C) und anschließend - nach wiederholtem Waschen - mit spezifischem anti-Lewis Y-Antikörper (human) oder Ziegen-anti-HE2-Antikörper (1 µg/ml gelöst in Verdünnungspuffer: 2%FCS in PBS; 100 µl) eine halbe Stunde bei 37°C inkubiert. Nicht gebundene Antikörper werden durch dreimaliges Waschen mit Waschpuffer entfernt. Die gebundenen Antikörper werden mittels HRP-Konjugat, welches spezifisch für den jeweiligen Detektions-Antikörper ist (Ziegen anti-human IgG+A+M HRP von Zymed (USA) für anti-Lewis Y-Antikörper; Maus anti-Ziegen IgG HRP (Axell, USA) für anti-HE2 Antikörper, 1 µg/ml, 100 µl), detektiert (30 Minuten bei 37°C). Nach anschließendem Waschen (3x mit Waschpuffer und 1x mit Färbepuffer) wird durch gebundenes HRP-Konjugat die Färbung von 100 µl Orthophenylendiamin-Dihydrochlorid-Lösung (Sigma, USA; gelöst in Färbepuffer und aktiviert it H₂0₂; 30%, Merck, Deutschland) eingeleitet und die Farbentwicklung wird mit 15% Schwefelsäure (50 µl) gestoppt. Auf einem Mikroplatten-Photometer (Labsystem, Modell Nr. 354) wird die entwickelte Extinktion bei 492 nm gemessen, die Referenzwellenlänge beträgt 620 nm.

Nach einem weiteren Waschschritt mit Färbepuffer (24,3 mM Zitronensäure, 51,4 mM Na₂HPO₄, pH5).

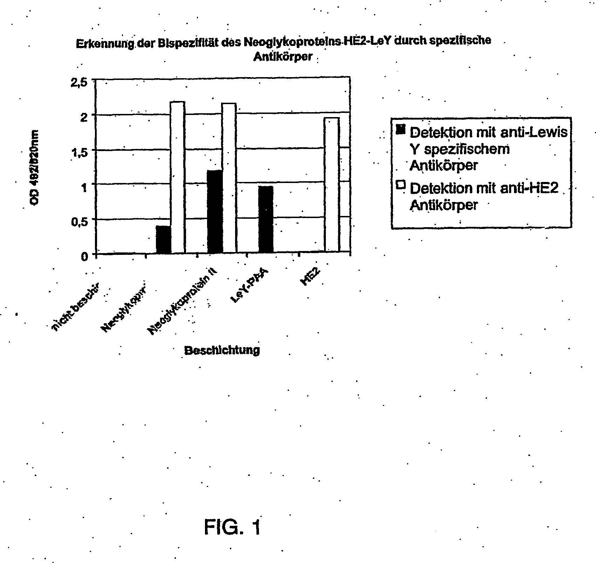

In Figur 1 sind die Ergebnisse dargestellt: Beide Neoglykoproteine haben beide Spezifitäten (HE2 und Lewis Y), Neoglykoprotein II ist stärker funktionell glykosyliert als Neoglykoprotein I und gibt daher ein höheres Signal mit dem anti-Lewis Y-Antikörper.

### Sandwich ELISA:

Humaner Anti-Lewis Y-Antikörper (10 µg/ml gelöst in Beschichtungs-Puffer; 100 µl) wird auf eine Mikrotiterplatte (Nunc, Maxisorb) unspezifisch gebunden (1 Stunde Inkubation bei 37°C), nach dreimaligem Waschen mit Waschpuffer (200 µl) mit 5% fötalem Kälberserum in PBS (200 µl) blockiert (30 Minuten bei 37°C inkubieren) und mit HE2-Lewis Y-Neoglykoprotein I und II sowie HE2 als Kontrolle in verschiedenen Konzentrationen (1,25 - 7,63x10⁻⁶ µg/ml; 100 µl) 1 Stunde bei 37°C inkubiert. Nach dreimaligem Waschen in Waschpuffer wird mit Ziegen anti-HE2-Antikörper (1 µg/ml in Verdünnungspuffer; 100 µl) 30 Minuten bei 37°C inkubiert. Uberschüssige Antikörper werden durch einen anschließenden Waschschritt (3x mit Waschpuffer) entfernt. Gebundene Antikörper werden durch Inkubation (30 Minuten, 37°C) mit Maus anti-Ziegen IgG HRP (Axell, 1:1000 in Verdünnungspuffer gelöst, 100 µl) erkannt: Nach anschließendem Waschen (3x mit Waschpuffer, 1x mit Färbepuffer) löst gebundenes HRP-Konjugat eine Färbereaktion von 100 µl zugesetzter Orthophenylendiamin-Dihydrochloridlösung (Sigma, 10 mg gelöst in 25 ml Färbepuffer und aktiviert mit 10 µl H₂O₂; 30%, Merck) aus. Die Farbreaktion wird mit 50 µl 15% H₂SO₄ gestoppt und die Extinktion bei 492 nm (Referenzwellenlänge 620 nm) auf einem Mikroplatten-Photometer gemessen (Labsystem, Modell Nr. 354).

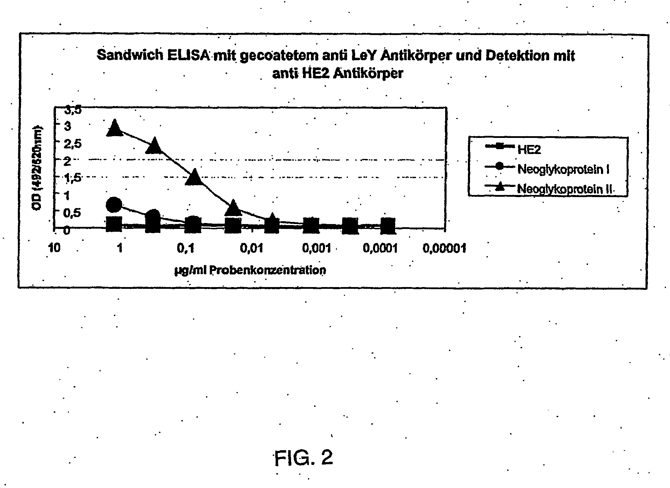

Aus Figur 2 kann man ersehen, dass beide Neoglykoproteine in diesem Sandwich-ELISA detektiert werden können, "Neoglykoprotein II" ist stärker glykosyliert, wird daher stärker vom vorgecoateten anti-Lewis Y-Antikörper zurückgehalten.

### SDS-PAGE:

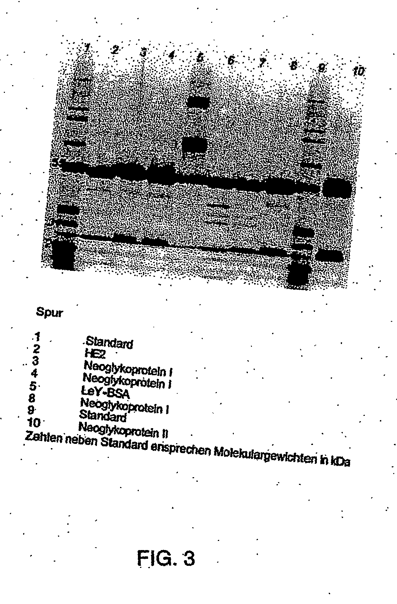

Die Proben (nicht konjugierter HE2-Antikörper, Neoglykoprotein I und II sowie Lewis Y-BSA) werden in reduzierendem Puffer hitzebehandelt (85°C, 2 Minuten) und auf einem Polyacrylamidgel (4-12% Bis-Tris-Gel) elektrophoretisch aufgetrennt. Die dadurch der Größe nach getrennten Proteine werden durch Silberfärbung sichtbar gemacht (NOVEX SDS-PAGE-System, Invitrogen, USA). Auf dem Gel ist nur eine sehr geringfügige Erhöhung des Molekulargewichtes durch die Glykosylierung mit Lewis Y-Tetrasaccharid bemerkbar (siehe Figur 3).

### Western Blot:

Wie beim SDS-PAGE werden die Proben der Größe nach getrennt. Anschließend werden die aufgetrennten Proteine auf eine Nitrozellulosemembran geblottet, eine Stunde in 3%igem Milchpulverlösung blockiert und anschließend zwei Stunden mit humanem anti-Lewis Y-Antikörper (10 µg/ml in PBS) inkubiert. Gebundene Antikörper werden mit Ziegen anti-human HRP-Konjugat (1:500 in PBS), welches spezifisch für den anti-Lewis Y-Antikörper ist, detektiert. Durch eine anschließende Färbereaktion werden die Lewis Y-glykosylierten Proteine sichtbar gemacht.

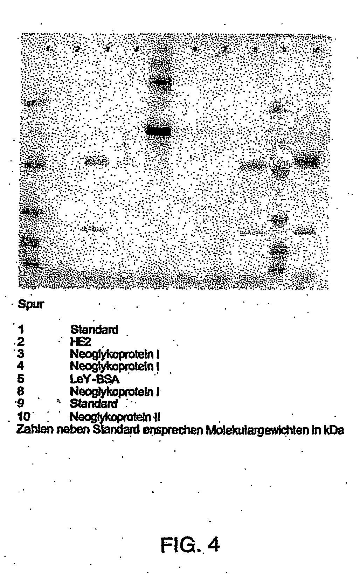

Wie aus Figur 4 ersichtlich, reagiert das Neoglykoprotein II mit dem anti-Lewis Y-Antikörper, Neoglykoprotein I scheint zu schwach glykosyliert zu sein, als dass es in diesem Test mit anti-Lewis Y detektiert werden könnte.

### Immunantwort gegen HE2 und Lewis Y:

Seren immunisierter Affen werden zu verschiedenen Zeitpunkten vor und nach der Immunisierung auf die Bildung einer humoralen Immunantwort gegen HE2 und gegen Lewis Y überprüft. Das Immunisierungsschema ist dabei folgendermaßen (die einzelnen Immunisierungen wurden subkutan durchgeführt: 500 µg Protein, adsorbiert auf 1,67 mg Aluminium-hydroxid in 0,5 ml 1 mM Phoshat-Puffer pH 6,0/155 mM NaCl).

### Immunisierungszeitpunkte:

| | |
|---|---|
| Tag 1 | (T1) |
| Tag 15 | (T15) |
| Tag 29 | (T29) |
| Tag 43 | (T43) |
| Tag 57 | (T57) |
| Tag 71 | (T71) |

### Blutabnahmen zur Serum-Gewinnung:

| | |
|---|---|
| Tag 1 | (T1) |
| Tag 15 | (T15) |
| Tag 29 | (T29) |
| Tag 43 | (T43) |
| Tag 57 | (T57) |
| Tag 71 | (T71) |
| Tag 92 | (T92) |

### HE2-ELISA:

HE2-Antikörperlösung wird in Beschichtungs-Puffer auf 10 µg/ml verdünnt und 1 Stunde bei 37°C inkubiert (100 µl). Nach dreimaligem Waschen mit Waschpuffer wird mit 200 µl 5 % fötalem Kälberserum in PBS 30 Minuten bei 37°C blockiert. Nach einem weiteren Waschschritt (wie zuvor) werden 100 µl von verschiedenen Verdünnungen der Seren immunisierter Tiere auf die Mikrotiterplatte aufgetragen (Verdünnungspuffer: 2% fötales Kälberserum in PBS) und 1 Stunde bei 37°C inkubiert. Durch dreimaliges Waschen mit Waschpuffer werden nicht gebundene Antikörper entfernt und anschließend 30 Minuten bei 37°C mit 100 µl Ziegen-anti-human- IgG+A+M HRP-Lösung (Zymed, 1:1000 verdünnt in Verdünnungspuffer) inkubiert. Nach dreimaligem Waschen mit Waschpuffer und einmaligem Waschen mit Färbepuffer wird durch gebundenes HRP eine Farbreaktion von Orthophenylendiamin-Dihydrochlorid (Sigma, 10 mg gelöst in 25 ml Färbepuffer aktiviert mit 10 µl H₂O₂, 30%, Merck) ausgelöst. Mit 50 µl Schwefelsäure (15%, Fluka) wird die Reaktion gestoppt und die Extinktion bei 492 nm (Referenzwellenlänge 620 nm) auf einem Mikroplatten-Photometer (Labsystems, Modell Nr. 354) gemessen.

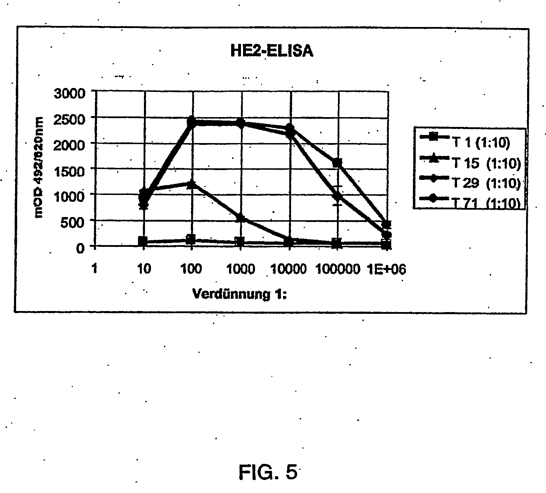

Figur 5 zeigt das Ergebnis des HE2-ELISA. Man sieht, dass die Immunantwort gegen das Trägerprotein bereits nach 2 Immunisierungen sehr stark ist.

Durch die Immunisierung eines Rhesusaffen mit Neoglykoprotein wird also eine starke humorale Immunantwort gegen HE2 induziert.

### LewisY-AA-ELISA_{:}

Lewis Y-PAA (Lectinity Holding, Inc. Bad Homburg, Germany) wird in Beschichtungspuffer auf 10 µg/ml verdünnt und 1 Stunde bei 37°C inkubiert (100 µl). Nach dreimaligem Waschen mit Waschpuffer wird mit 200 µl 5% fötalem Kälberserum in PBS 30 Minuten bei 37°C blockiert. Nach einem weiteren Waschschritt (wie zuvor) werden 100 µl von verschiedenen Verdünnungen der Seren immunsierter Tiere auf die Mikrotiterplatte aufgetragen (Verdünnungspuffer: 2% fötales Kälberserum in PBS) und 1 Stunde bei 37°C inkubiert. Durch dreimaliges Waschen mit Waschpuffer werden nicht gebundene Antikörper entfernt und anschließend 30 Minuten bei 37°C mit 100 µl Ziegen-anti-human IgG+A+M HRP-Lösung (Zymed, 1:1000 verdünnt in Verdünnungspuffer) inkubiert. Nach dreimaligem Waschen mit Waschpuffer und einmaligem Waschen mit Färbepuffer wird durch gebundenes HRP eine Farbreaktion von Orthophenylendiamin-Dihydrochlorid (Sigma, 10 mg gelöst in 25 ml Färbepuffer aktiviert mit 10 µl H₂O₂, 30%, Merck) ausgelöst. Mit 50 µl Schwefelsäure (15%, Fluka) wird die Reaktion gestoppt und die Extinktion bei 492 nm (Referenzwellenlänge 620 nm) auf einem Mikroplatten-Photometer (Labsystems, Modell Nr. 354) gemessen.

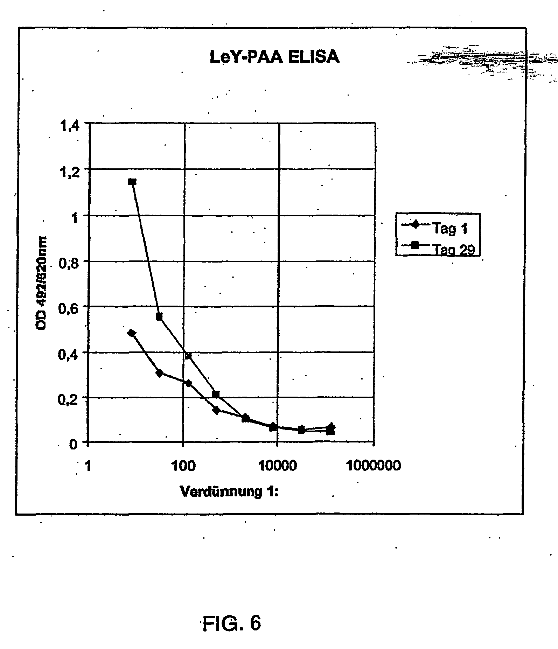

Figur 6 zeigt, dass durch die Immunisierung eines Rhesusaffen mit Neoglykoprotein eine spezifisch gegen Lewis Y gerichtete humorale Immunantwort ausgelöst wird.

### Beispiel 2: Kopplung des SialylTn Kohlehydrats an HE2

SialylTn-O(CH₂)₃NH(CH₂)₄COO-pNp wurde an HE2 gekoppelt.
Das Endprodukt wurde mittels SEC, LDS-PAGE, Western Blot und verschiedenen ELISA Tests analysiert.

### Methoden

### Material

HE2 Panorex, 10mg/ml, Lot170901
SialylTn-O(CH₂)₃NH(CH₂)₄COO-pNp, 2x5mg, Fa. Lectinity
DMF (N,N-Dimethylformamid (anhydrous, Merck))
Kopplungspuffer: 0,1m Na₂HPO₄ + 0,15M NaCl (pH=8)
Formulierungspuffer: NaCl 0,86% + 1mM Na₂HPO₄ (pH=6,0)

### Verfahren

1. 100mg HE2 (V=10ml; Konz:10mg/ml) wurden gegen 2x 700ml Kopplungspuffer dialysiert unter Verwendung einer Slide-A-Lyzer Dialysekassette bei 4°C für 20 Stunden, Auffüllen des Volumens auf ~10ml, die Konzentration gemäß SEC betrug ~10mg/ml.
2. 2× 5mg SialylTn-O(CH₂)₃NH(CH₂)₄COO-pNp wurden mit 2x 100µl DMF (100µl/Röhrchen) gelöst.
3. Die Lösung des SialylTn (in DMF) wurde auf ~10ml (~100mg) mit eisgekühltem HE2 (in Kopplungspuffer) aufgefüllt.
4. Beide SialylTn-Vials wurden mit 100µl DMF gewaschen (es erfolgte ein Transfer von Röhrchen 1 auf Röhrchen 2), dieses wurde der Reaktionsmischung ebenfalls zugegeben.
5. Die Reaktionsmischung wird über Nacht rotieren gelassen (28 Stunden) bei +4°C. Die Reaktionskinetik wurde mit SEC geprüft.
6. Die Endlösung des HE2-SialylTn (10ml, ~10mg/ml) wurde gegen 2x 800ml Formulierungspuffer dialysiert unter Verwendung einer Slide-A-Lyzer Dialysekassette bei 4°C für 20 Stunden.

### Analyse:

### Größenausschlusschromatographie:

Die Konzentrationen an HE2-SialylTn wurden quantifiziert durch Größenausschlusschromatographie (SEC) auf einer ZORBAX GF-250 Säule in einem Dionex System. Das HPLC System wurde mit einem Gelfiltrationsstandard getestet. (Fa. BioRAD).

HE2 wurde als Referenzstandard für die Quantifizierung des HE2-SialylTn gewählt. Der Abfall der Retentionszeit (korreliert mit dem Anstieg des Molekulargewichts)korreliert mit der Effektivität der Kopplungsreaktion des SialylTn an HE2. Die erhaltenen Daten zeigen, dass die Kopplungseffizienz mit der Reaktionszeit ansteigt und eine Sättigung bei 23-27 Stunden erreicht wird.

### LDS-PAGE (Lithium Dodecylsulfat PAGE)

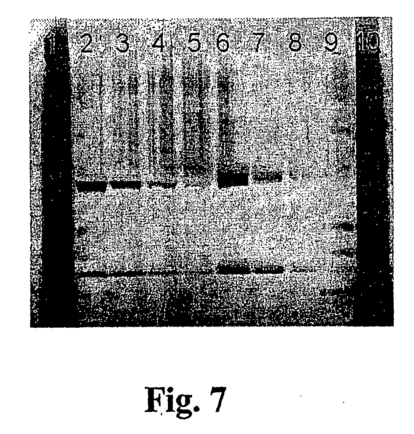

LDS-PAGE mit Bis-Tris-Gel (4-12%)
SilverXpress^{™}-Färbung: siehe "NuPAGE Bis-Tris-Gel" Instruction Booklet, Seite 13
Die Ergebnisse sind in Fig. 7 dargestellt.

| **lane** | **Probe** | **Konz.** | **Volumen [**µ**l]** | **Präparation** |
|---|---|---|---|---|
| 1 | Mark 12 MW Standard | - | 10 | keine |
| 2 | HE2 dialysiert in Kopplungspuffer | 20µg/ml | 10 | s. SOP |
| 3 | HE2 dialysiert in Kopplungspuffer | 10µg/ml | 10 | s. SOP |
| 4 | HE2 dialysiert in Kopplungspuffer | 50µg/ml | 10 | s. SOP |
| 5 | HE2 dialysiert in Kopplungspuffer | 2,5µg/ml | 10 | s. SOP |
| 6 | HE2SiaTn dial. in Formulierungsungspuffer | 20µg/ml | 10 | s. SOP |
| 7 | HE2SiaTn dial. in Formulierungsungspuffer | 10µg/ml | 10 | s. SOP |
| 8 | HE2SiaTn dial. in Formulierungsungspuffer | 5µg/ml | 10 | s. SOP |
| 9 | HE2SiaTn dial. in Formulierungsungspuffer | 2,5µg/ml | 10 | s. SOP |
| 10 | Mark 12 MW Standard | - | 10 | keine |

Fig. 7: Im Vergleich zu HE2 (lanes 2-5) erfolgt im HE2-SialylTn Kopplungsprodukt (lanes 6-8) ein deutlicher Anstieg im Molekulargewicht der schweren Kette was darauf hindeutet, dass SialylTn erfolgreich an die schwere Kette (50 kDa) des HE2 Antikörpers gekoppelt wurde. Zusätzlich deutet das Auftereten einer zweiten Bande (mit geringfügig unterschiedlichem Molekulargewicht) zur 25 kDa Bande darauf hin, dass auch die leichte Kette teilweise an SialylTn gekoppelt wurde.

### Western Blot

Western Blot mit Kaninchen x Maus IgG2a

### Verfahren:

**1.** LDS-Gel mit Bis-Tris-Gel (4-12%)
2. Western Transfer Anweisungen siehe NuPAGE Bis-Tris-Gel Instruction Booklet Seite 14-20 (mit Immobilon Transfer Membran PVDF 0,45µm, Fa. Millipore)
3. Membran Entwicklung:

### Material:

Konjugat: Kaninchen x Maus IgG2a - HRP, #61-0220, Fa. Zymed Färbelösung 1: 15mg HRP-Farbreagens (Fa. BioRAD) in 5ml MetOH Färbelösung 2: 15µl 30%H₂O₂ in 25ml PBS def. 1x

### Verfahren:

Blockieren der Membran mit 3% Trockenmilchpulver in PBS für 1h bei RT
Waschen der Membran mit PBS
Inkubieren mit dem Konjugat (Verdünnung 1:1000 in PBS) für 1h bei RT
Waschen der Membran mit PBS
Entwicklung mit Färbelösungen 1+2 und stoppen der Färbung mit Wasser.

Western Blott mit anti SialylTn CD175s (IgG type) / Ratten x Maus IgG1 - HRP.

### Verfahren:

1. LDS-PAGE Gel mit Bis-Tris-Gel (4-12%)
2. Western Transfer: Instruktionen siehe "NuPAGE Bis-Tris-Gel" Instruction Booklet, Seite 14-20 (Verwendung einer Immobilon Transfer Membran PVDF 0,45µm, Fa. Millipore)
3. Membran Entwicklung:

### Material:

Sekundärer Antikörper: anti-SialylTn CD175s (IgG type), 90µg/ml, Fa. DAKO, Code.Nr. M0899, Lot. 089(601)
Konjugat: Ratte x Maus IgG1 - HRP, Fa. Becton Dickinson, Mat.Nr. 559626, Batch: 37205
3% Trockenmilchpulver in PBS def1x
Färbelösung 1: 15mg HRP-Farbreagens (Fa. BioRAD) in 5ml MetOH
Färbelösung 2: 15µl 30%H₂O₂ in 25ml PBS

### Verfahren:

Blockieren der Membran mit 3% Trockenmilchpulver in PBS für 1h bei Raumtemperatur (RT)
Waschen der Membran mit PBS
Inkubieren mit sekundärem Antikörper, (Konzentration 10µg/ml) V=5ml, für 1h bei RT
Waschen der Membran mit PBS
Inkubieren mit Konjugat (verdünnt 1:1000 in PBS) für 1h bei RT Waschen der Membran mit PBS
Entwickeln mit Färbelösungen 1+2 und stoppen der Reaktion mit Wasser.

Der Anstieg im Molekulargewicht der schweren Kette des HE2 Antikörpers nach Kopplung mit SialylTn wurde durch den Western Blot and die Färbung mit einem Kaninchen anti Maus IgG2a-HRP bestätigt.

Ein Standard ELISA wurde durchgeführt um zu zeigen, wie viel der anti-idiotypischen Bindungsaktivität (des HE2) im Kopplungsprodukt erhalten bleibt.

Immobilisierter IGN111 bindet den antiidiotypischen HE2 der durch einen anti Maus IgG2a-HRP erkannt wird.

Es wurde gezeigt, dass HE2 etwa 2-3 mal reaktiver ist als HE2-SialylTn, was bedeutet, dass nur ein geringfügiger Verlust an Bindungskapazitäten nach der Kopplung auftritt.

Ein weiterer Standard ELISA wurde durchgeführt um SialylTn durch einen Maus anti-SialylTn-Antikörper zu detektieren. Dazu wurde das Ausgangsmaterial HE2 und das Kopplungsprodukt HE2-SialylTn immobilisiert. Zum Nachweis des SialylTn wurden anti-SialylTn (Maus IgG)/Ratte anti Maus IgG1-HRP verwendet.

Die Ergebnisse zeigen, dass das HE2-SialylTn Reaktionsprodukt tatsächlich SialylTn Gruppen trägt im Gegensatz zu HE2 vor der Kopplung.

### Zusammenfassung:

SialylTn wurde erfolgreich an den HE2 Antikörper gekoppelt. Die Kopplungsreaktion weist eine verlängerte Zeit der Reaktionskinetik auf, wobei die Sättigung nach etwa 24 Stunden erreicht wird. SialylTn wurde vorwiegend an die schwere Kette des HE2 Antikörpers gekoppelt, wohingegen die leichte Kette nur teilweise mit SialylTn gekoppelt wurde.

Das HE2-SialylTn Kopplungsprodukt behält den Großteil der idiotypischen Spezifität des HE2 bei, der SialylTn Teil dieses Neoglykoproteins wird durch SialylTn spezifische Antikörper erkannt.

Diese Ergebnisse zusammen deuten klar darauf hin, dass die antigenischen Epitope beider, sowohl der HE2 Proteinteil als auch der SialylTn Teil in der Multiepitop-Vakzine konserviert sind. Der Endotoxin-Gehalt liegt unter der Nachweisgrenze.

### Beispiel 3: Formulierung des HE2-SialylTn unter Verwendung verschiedener Adjuvantien:

1.Formulierungspuffer (NaCl, Na₂HPO₄), Thimerosal, Alhydrogel
2.Formulierungspuffer, Thimerosal, Alhydrogel, LPS, E. coli (Sigma, No.L-4391).

### Beispiel 4: Ergebnisse der Immunisierung von Rhesusaffen mit dem HE2-SialylTn Neoglykoprotein: Toleranz und Immunogenizitätsstudien

### Immunisierungsschema und Blutabnahmen

Rhesusaffen wurden vier Mal durch subkutane Immunisierungen mit 500 µl der Vakzine (enthalten 500µg HE2, adsorbiert an Alhydrogel in 1mM Na-Phosphat Puffer, pH=6.0, supplementiert mit 0,86% NaCl) geimpft.

Die Blutabnahmen erfolgten an den Tagen -3, 1, 8, 15, 29, 57 und 71. Das Blut wurde geronnen lassen (SST Clotactivator-Röhrchen (Vacutainer)); Serum wurde in Nunc Röhrchen übergeführt 1,8ml (375418);

| Tag | Datum | Immunisierung | Blutabnahme |
|---|---|---|---|
| -3 | | nein | ja |
| 1 | | 500µl | ja, vor der Imm. |
| 8 | | nein | ja |
| 15 | | 500µl | ja, vor der Imm. |
| 29 | | 500µl | ja, vor der Imm. |
| 57 | | 500µl | ja, vor der Imm. |
| 71 | | | ja |

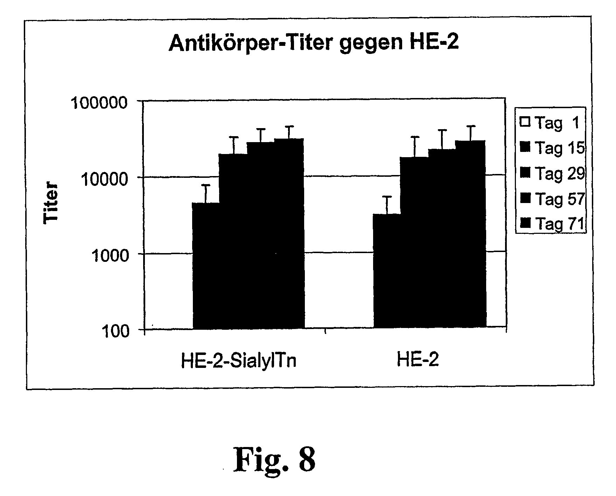

Fig. 8 zeigt die Ergebnisse der Immunisierungsstudien in Rhesusaffen. Die Induktion der Immunantwort gegen HE2 durch die HE2-SialylTn Multiepitop-Vakzine ist vergleichbar mit der Immunantwort die durch HE2 induziert wird.

### ELISA

Präserum (Tag 1) und Immunserum (Tag 15,29,57,71) wurden im Hinblick auf die Immunantwort gegen das immunisierende Antigen (HE2) durch einen HE2 ELISA und SialylTn ELISA analysiert. Ein Zigen anti Human IgGAM-HRP (Zymed, Nr.62-8320, Lot. 20571004) Konjugat wurde für den Nachweis verwendet.

Der SialylTn ELISA wurde ähnlich wie der LewisY ELISA durchgeführt, abgesehen von einigen Modifikationen. Zusammenfassend wurden ELISA Platten (F96 Maxisorp Mikrotiter Platten, NUNC) mit 20µg/ml SialylTn-PAA (30%mol, Syntesome) beschichtet, verdünnt in Beschichtungspuffer für die Dauer von 2h bei 37°C. Nach dem Waschschritt (drei Mal, 1:10 verdünnte WBK) wurden die ELISA Platten mit 5% FCS in PBS geblockt (30min., 37°C), gefolgt von einem nächsten Waschschritt. Die Proben (vorverdünnt in 2% FCS) wurden für 1h bei 37°C inkubiert. NAS (NA-Pool 25-07-01, Fa. Biotest) und PBS wurden als negative Kontrollen verwendet. Für den SialylTn ELISA wurde ein Maus anti-SialylTn CD175s Antikörper (DAKO, Code Nr. M0899, Lot. Nr. 039(601) mit einer Ausgangskonzentration von 20µg/ml verwendet, der als Positivkontrolle diente.

Nach einem neuerlichen Waschschritt wurden die Platten mit einem Ziegen anti-human Ig (H+L)-HRP Konjugat (1:4000, SB, Southern Biotechnology Cat.Nr. 2010-05, Lot.Nr. L262-S496L) oder einem Maus anti-human IgG (Fc)-HRP Konjugat (1:1000, SB, Cat.Nr. 9040-05, Lot.Nr. J560-NC21G) oder einem Maus anti-human IgM-HRP Konjugat (1:1000, SB, Cat.Nr. 9020-05, Lot.Nr. H018-W089) bei 37°C für 30min inkubiert. Für den Nachweis des Maus anti-SialylTn Antikörpers (positive Kontrolle) wurde ein Kaninchen anti-Maus IgG1-HRP (Zymed, Nr. 61-0120, Lot.Nr.00761146) verwendet.

Nach dem nächsten Waschschritt wurde das Substrat OPD (1 OPD Tablette gelöst in 25ml Färbepuffer, + 10µl 30% H₂O₂) zugegeben. Nach 10min. wurde die Färbereaktion durch Zugabe 50µl H₂SO₄ (30%) gestoppt.

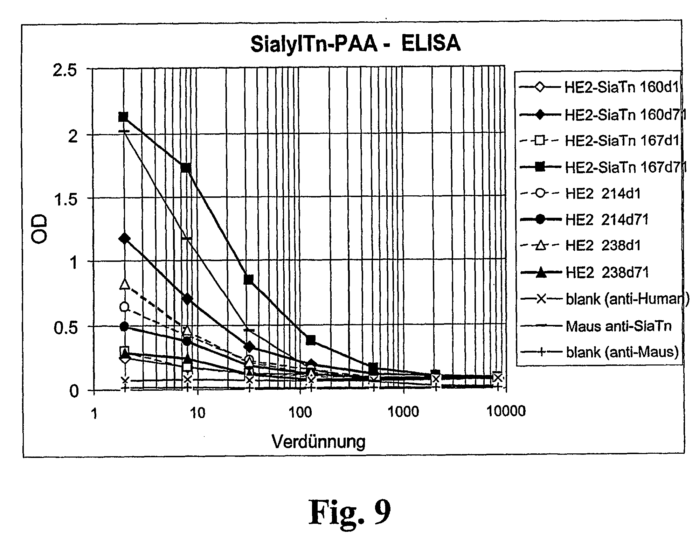

Fig.9 zeigt die Ergebnisse des SialylTn-PAA ELISA. Die Induktion der Immunantwort gegen das immunisierende Antigen HE2 und das Zielantigen EpCAM ist vergleichbar mit der ursprünglichen HE2 Einzelepitop-Vakzine. Es erfolgte also eine Induktion der Immunantwort gegen das Kohlehydratantigen SialylTn, diese Immunantwort wurde bei der Vakzinierung durch die HE2 Einzelepitop-Vakzine nicht induziert.

### Affinitätschromatographie

Eine Affinitätschromatographie wurde auf einem ÄKTA-Explorer, Pharmacia FPLC System durchgeführt. 1 ml des Serums (Präserum or Immunserum) wurde 1:10 mit PBS 1x + 0,2M NaCl, pH=7,2 (=Puffer A) verdünnt. Nach der Säulenäquilibrierung wurde das verdünnte Serum auf die Chromatographiesäule aufgetragen mit einer Flussrate von 1ml/min. Ungebundene Probe wurde mit einem Puffer A heruntergewaschen bis die UV-Linie (280nm) unterhalb 5mAU lag. Die Elution der gebundenen Probe wurde schrittweise was mit Glycin-Puffer pH=2,9 (=Puffer B, Elutionspuffer) durchgeführt. Die gewünschten Fraktionen wurden sofort mit 1M NaHCO₃ neutralisiert und durch Zugabe von Natriumazid (Endkonzentration: 0,02%) stabilisiert.

Folgende Affinitätschromatographiesäulen wurden verwendet:
1. **HE2-Sepharose:** HE2 gekoppelt an CH-Sepharose 4B Säule, Lot 20000905-070301 (SS LJ5/174)
2. **EpCAM-Sepharose:** EpCAM gekoppelt an CH-Sepharose 4B Säule (IF LJ32/54+57)
3. **HE2-SialylTn-Sepharose:** HE2-SialylTn gekoppelt an CH-Sepharose 4B Säule

Die Reinigung des Prä- oder Immunserums wurde entweder durch a) Einzelschritt-Affinitätschromatographie, oder b) sequenzielle Affinitätschromatographie mit dem Eluat der ersten Säule, das auf eine zweite Affinitätschromatographie-Säule aufgetragen wurde, durchgeführt oder c) Differential-Affinitätschromatographie mit dem Durchlauf der ersten Säule die auf eine zweite Affinitätschromatographie geladen wurde.

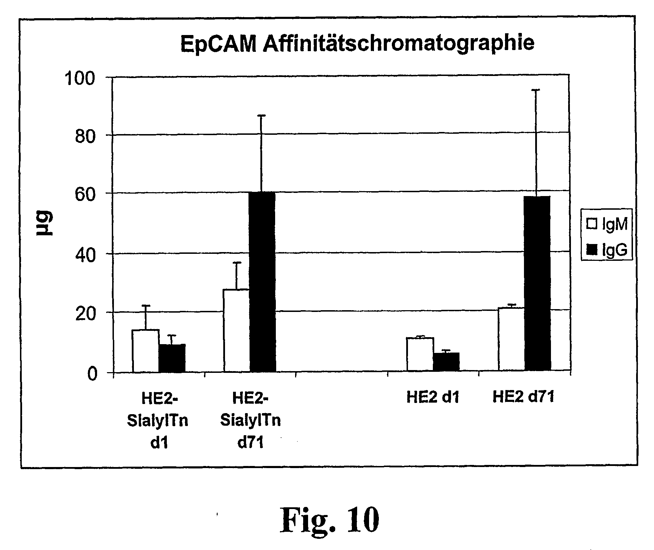

Nach der Quantifizierung der Mengen an Immunoglobulinen durch SEC, wurden die verbleibenden Serumeluate durch die Zugabe von FCS (Endkonzentration 2%) stablisiert und bei +4°C aufbewahrt. Results are shown in Fig.10

### Größenausschlußchromatographie:

Die Mengen an Immunoglobulinen (IgG, IgM) wurden durch Größenausschlusschromatographie (SEC) auf einer ZORBAX GF-250 Säule auf einem DIONEX-System quantifiziert. Das HPLC System wurde durch einen Gelfiltrationsstandard getestet.

Für die Quantifizierung der Mengen an Immunoglobulinen in den ÄKTA Eluaten, wurde eine Standardkurve von humanem IgG (Sandoglobulin) in einem Bereich von 1,95 - 25 µg/ml vorbereitet und als Referenzstandard verwendet.

### Zellinien

WM9, SKBR5, KATOIII, HT29, and OVCAR3 Zellen wurden in RPMI1640 Medium mit L-Glutamin versetzt mit 10% FCS und 1% Penicillin/Streptomycin kultiviert. CT26 und CT26-KSA (Klon #21 Sp1-3; EpCAM transfizierte CT26 cells) wurden in DMEM versetzt mit 10 % FCS, 1% nicht-essentiellen Aminosäuren, 1% Natriumpyruvat, 1% Vitamin, L-Glutamin und 1% Penicillin/Streptomycin wachsen gelassen.

### FACS Analyse

Zellen wurden in PBS Puffer enthaltend 0,2 mg/ml EDTA geerntet. Kultivationsmedium wurde zu den abgelösten Zellen zugegeben, diese werden anschließend pelletiert und zweimal in FACS Puffer gewaschen(PBS Puffer versetzt mit 2 % FCS und 0,1 % NaN₃). 10.000 Zellen wurden mit PBS enthaltend 10 % FCS und 0,1 % Natriumazid für 30 Minuten auf Eis blockiert. Nach Übertragen der Zellen in FACS Puffer, wurden die Zellen mit den Eluaten aus der Affinitätschromatographie des Präserums oder des Immunserums für eine Stunde auf Eis inkubiert. Als Positivkontrollen wurden die folgenden primären Antikörper verwendet: IGN311 (EN25.888) für die LewisY Färbung, und HE2 und KS1/4 für die EpCAM Färbung. Die Zellen wurden zweimal in FACS Puffer gewaschen und mit den Nachweisantikörpern unter Lichtschutz für 30 Minuten inkubiert (Schaf anti-human IgGAM-FITC (gamma-und Leichtketten-spezifisch), Silenius, Verdünnung 1:1000 oder Kaninchen anti-Maus IgGAM F(ab)₂'-FITC Dako (gamma und Leichtketten-spezifisch), Verdünnung 1:100, für den Nachweis des murinen HE2 and KS1/4 Antikörper. Nach dreimaligem Waschen in FACS Puffer, wurden die Fluoreszenzintensitäten (10 000 Zellen in 100 µl FACS Puffer pro Analyse) mit einem FACS-Calibur System (Becton Dickinson) gemessen.

Kontrollfärbung mit:
IGN311 (25µg/ml), KS1/4 (1µg/ml), HE2 (1µg/ml), SKBR5, KatoIII, WM9, CT26 und CT26KSA Zellen.

### Ergebnisse

### Immunantwort gegen immunisierende Antigen(HE-2)

Präserum (Tag 1) or Immunserum (Tag 15,29,57,71) aller Tiere wurde im Hinblick auf die Immunantwort gegen das immunisierende Antigen (HE2) durch HE2 ELISA analysiert. Ein deutlicher Immunisierungseffekt wurde in allen geimpften Gruppen gefunden, wobei die Stärke der Immunantwort als Funktion der Zeit (und der Zahl der Immunisierungen) anstieg. Wichtig war, dass keines der Adjuvantien den HE2 Titer steigerte oder die Kinetik der Immunantwort im Vergleich zur Kontrollgruppe die das Antigen ohne Adjuvans erhalten hatte (P6/01) ansteigen ließ. Die Multiepitop HE2-SialylTn Vakzine (P2/01) induzierte einen HE2 Titer der vergleichbar war mit der der HE2 Vakzine (P6/01).

Die Ergebnisse sind in Fig. 8 zu sehen, wobei es sich hier um eine HE-2 Rhesusaffen Studie handelt.

Die HE2-SialylTn Multiepitop Vakzine induziert eine Immunantwort gegen das zweite Antigen, SialylTn in allen immunisierten Tieren, wie dies im SialylTn ELISA nachgewiesen wurde, ein Effekt, der nicht nach der Impfung mit der HE2 Einzelepitop-Vakzine gefunden wurde.

### Reinigung des Serums durch direkte EpCAM Affinitätschromatographie:

Immunseren (Tag 71) wurden durch direkte EpCAM Affinitätschromatographie gefolgt von einer Größenausschlußchromatographie (SEC) analysiert. In allen geimpften Gruppen wurden signifikante Mengen Ig (IgG und IgM) im Immunserum gefunden (60-87µg Ig) was wesentlich höher war als im Vergleich zum Gehalt der Ig im Präserum (13-22µg). Weiters konnte ein IgG switch nach der Impfung gesehen werden, mit erhöhten IgG/IgM Verhältnissen im Immunserum. Hingegen führten die Adjuvantien zu keinem Anstieg der Ig (IgG, IgM), die eine spezifische Reaktivität mit rEpCAM in den Immunseren aufweisen, wie dies durch die direkte EpCAM Chromatographie angenommen wurde im Vergleich zur HE2 Kontrollgruppe.

### SialylTn ELISA

Präserum (Tag 1) und Immunserum (Tag 71) der Multiepitop Vakzine (P2/01, HE-2-SialylTn) Gruppe im Vergleich zur P6/01 Kontrollgruppe wurden auf die Immunantwort gegen das SialylTn Kohlehydrat Antigen durch einen SialylTn-ELISA getestet.

Ein deutlicher Immunisierungseffekt, nämlich die Induktion der anti SialylTn Antikörper Titer, wurde in allen 4 immunisierten Tieren der P2/01 Gruppe gefunden, im Gegensatz dazu erfolgte kein Anstieg des SialylTn Antikörpertiters in der HE2 Kontrollgruppe nach der Immunisierung.

Die Ergebnisse sind in Fig. 8 dargestellt, die Induktion der Immunantwort gegen das immunisierende Antigen (HE2) und das Zielantigen (Ep-CAM) ist ähnlich wie bei der HE2 Einzelepitop-Vakzine. Die immunantwort gegen das Kohlehydratantigen SialylTn wird durch die Multiepitop-Vakzine induziert, diese Induktion wird nicht nach Immunisierung mit der HE2 Einzelepitop-Vakzine gefunden (P6/01)

### Beispiel 5: Herstellung eines rekombinanten Maus IgG2a HE-2 Antikörpers (rHE-2)

### Molekularbiologische Konstrukte

Der bicistronische pIRES Expressionsvektor von Clontech Laboratories Inc., Palo Alto, USA erlaubt, zwei Gene auf einem hohen level zu exprimieren und ermöglicht die Translation von zwei aufeinanderfolgenden offenen Leserahmen von der messenger RNA. Um positive Transformanten unter Verwendung eines Reporter Gens zu selektieren, wurde die interne Ribosomen Eingangsstelle (ribosome entry site (IRES)) in diesem Expressionsvektor trunkiert wodurch geringere Expressionsraten in diesem zweiten Leserahmen ermöglicht wurden.

Um dies zu erreichen, mußte die ursprüngliche IRES Sequenz reetabliert werden um unsere Anforderungen für die Expression der schweren und leichten Antikörperkette auf nahezu demselben Expressionsmenge zu ermöglichen.

Die attenuierte IRES Sequenz wurde für die Expression unserer Selektionsmarker verwendet.

Die DNA Manipulationen wurden entsprechend Standardverfahren durchgeführt. Unter Verwendung der PCR Technologie und des Advantage-HF PCR Kits (CLONTECH laboratories Inc., Palo Alto, USA), wurden die schwere und leichte Kette des HE2 Antikörpers amplifiziert. Es wurden Primer Sequenzen verwendet, um erstens die gewünschten Restriktionsschnittstellen einzuführen, die für den Einbau des Gens in die Expressionsvektoren notwendig sind und zweitens wurden KOZAK Sequenzen upstream der offenen Leserahmen eingebaut.

The autologen Signalsequenzen wurden verwendet um die nackten Polypeptidketten in den sekretorischen Kreislauf zu dirigieren. Die Primer wurden von MWG-Biotech AG, Deutschland erworben. Eine Zweischritt-Klonierungstechnologie wurde entwickelt: Die Kappa-Kette enthaltend ihre autologe Signalsequenz wurde als *Xho I,* Mlu I Fragment amplifiziert und in den Expressionsvektor ligiert unter Verwendung des "Rapid ligation kits" (Roche, Deutschland) entsprechend den Anweisungen des Herstellers. Ein chemisch kompetenter E. coli Bakterienstamm DH5alpha (Gibco BRL) wurde mit dem Konstrukt transfiziert, und unter Verwendung eines *Ampicillin* Selektionsmarkers amplifiziert. In einem zweiten Schritt wurde die rekonstruierte IRES Sequenz und die Gamma Kette, enthaltend auch die autologe Signalsequenz, amplifiziert als *Mlu I*, *Nco I* und *Nco I*, *Sal I* Fragmente und in einer Einzelschritt-Ligationsreaktion in den modifizierten Expressionsvektor, der bereits die HE2 Kappa Kette enthölt, ligiert. Dieses Konstrukt wurde amplifiziert unter Verwendung des E. coli Bakterienstamms DH5alpha (Gibco BRL). 25 Konstrukte, die aus verschiedenen PCR Proben abstammten, wurden mit den Restriktionsendonukleasen EcoRI und BamHI verdaut. Jene Konstrukte, die das korrekte Restriktionsmuster zeigten, wurden bidirektional sequenziert. In diesem Expressionskonstrukt wurde die unten beschriebene Selektionskassette eingebaut. Der Selektionsmarker DHFR wurde als PCR Xba I / Not I Fragment aus dem pSV2-dhfr Plasmid (ATCC #37146) amplifiziert. PCR-Primer führten diese Restriktionsschnittstellen ein. Die attenuierte IRES at. Sequenz wurde durch PCR aus pSV-IRES (Clontech #6028-1) als Sal I / Xba I Fragment amplifiziert. In einer Einzelschritt-Ligationsreaktion wurden IRES at. und DHFR in das bereits beschriebene Expressionskonstrukt ligiert, und zwar nach Verdau mit den entsprechenden Restriktionsendonukleasen und einem weiteren Dephosphorylierungsschritt.

Nach einer Transfektion den E. coli Bakterienstamm DH5alpha (Gibco BRL) wurden positive Transformanten durch PCR gescreent. Die Konstrukte wurden bidirektional sequenziert und für weitere Transfektionen von eukaryontischen zellen verwendet.

### Beispiel 6: Transfektion

Der charakterisierte eukaryontische Stamm, CHO (ATCC-CRL9096), wurde mit dem oben beschriebenen Expressionsvektor transfiziert. Der DHFR Selektionsmarker wurde dazu verwendet um stabile Zelllinien zu etablieren, die rHE-2 exprimieren. In einer 6-Näpfchen Zellkulturplatte wurde die Zellinie bei Zelldichten von 10⁵ Zellen in 2 ml complete Iscove's modifiziertem Dulbecco's Medium mit 4 mM L-Glutamin eingestellt auf den Gehalt von 1.5 g/L Natriumbicarbonat and versetzt mit 0.1 mM Hypoxanthin und 0.016 mM Thymidin, 90%; fötales Kälberserum, 10% (Gibco.BRL) ausgesät. Die Zellen wurden wachsen gelassen bis zu einer 50% Zelldichte. Die Zellen wurden dann transfiziert entsprechend der Anweisungen der Hersteller in Abwesenheit von Serum 2 µg DNA unter Verwendung des Lipofectin^{®} Reagens (Gibco-BRL). Die Transfektion wurde durch Zugabe von Vollmedium nach 6 oder 24 Stunden gestoppt.

### Beispiel 7: Selektion von positiven Transformanten und Kultivierung

Vollmedium wurde 24 oder 48 Stunden nach Transfektion durch Selektionsmedium ersetzt. Das FCS im Vollmedium wurde durch dialysiertes FCS (Gibco.BRL, Ursprung: Südamerika) ersetzt. 10 Tage nach der Selektion erschienen positive Transformanten als rasch wachsende multizelluläre Komglomerate. Die Konzentration des rHE-2 wurde in den Überständen durch spezifische Sandwich ELISA analysiert, die sowohl die variablen als auch die konstanten Domänen des Antikörpers erkennen. Jene Zellen, die eine hohe Produktivität zeigten, wurden 1:10 geteilt und in 75 cm² Zellkulturflaschen zur Aufbewahrung in flüssigen Stickstofff gegeben. Parallel dazu wurden diese Produzenten einem ansteigenden Selektionsdruck unterworfen indem Methothrexat dem Kulturmedium zugegeben wurde und die Zellen in einer 6 Näpfchen Zellkulturplatte ausgesät wurden. Das Verfahren wurde ungefähr zwei Wochen später nochmals wiederholt wenn die Zellen eine stabile Wachstumskinetik erreicht haben. Ausgehend von einer Konzentration von 0.005 µM, wurde die MTX Konzentration bei jeder Selektionsrunde verdoppelt bis zu einer Endkonzentration von 1.280 µM MTX und parallel dazu wurde in 96-well Zellkulturplatten subkultiviert. Die Überstände wurden wöchentlich durch einen spezifischen Sandwich ELISA getestet, der sowohl die variable als auch die konstante Domäne des Antikörpers erkennt. Stabile Kulturen mit der höchsten Produktivität wurden in 75-cm² Zellkulturflaschen übergeführt und schrittweise in 860-cm² rolling Zellkulturflaschen in nicht-selektives Medium übergeführt. Die Überstände wurden geerntet, zentrifugiert, analysiert und zur weiteren Reinigung geführt.

### Beispiel 8: Analyse der Expressionsprodukte

Die Überstände wurden mit einem spezifischen Sandwich ELISA getestet, der sowohl die variable als auch die konstante Domäne des Antikörpers erkennt. Der polyklonale, antiidiotypische Antikörper IGN111 wurde mit einer Konzentration von 10 µg/ml auf Maxisorp^{®} (NUNC) Adsorptionsplatten beschichtet. Der Antikörper wurde in Ziegen gebildet, die mit HE2 Fragmenten immunisiert wurden und der durch ein zweistufiges Chromatographie-Verfahren durch Affinität extrahiert wurde. Antikörper gegen die konstanten Regionen von Maus wurden in einem ersten Schritt auf eine polyklonale Maus IgG Säule adsorbiert, anti-idiotypische Antikörper wurden in einem zweiten Schritt durch Affinität an einer HE2 Agarose Säule gefangen. Das Endprodukt, die polyklonale IGN111 Antikörper Präparation erkennt daher die variable Domäne des HE2 Antikörpers. Die verbleibenden aktiven Gruppen wurden durch Inkubation mit

1% Trockenmilch blockiert und die Überstände wurden aufgetragen. Die exprimierten Antikörper wurden durch ihre konstanten Regionen über Kaninchen anti-Maus IgG2a-HRP Konjugate (Biozym) detektiert. Die Quantifizierung erfolgte durch vergleich mit einem auch auf die Säule geladenen und charakterisierten HE2 Standard Hybridom Antikörper.

Die Größendetermination der exprimierten Proteine erfolgte durch SDS-Polyacrylamidgelelektrophorese unter Verwendung von 4-14 % Acrylamid Gradientengelen in einer Novex^{®} (Gibco-BRL) Elektrophoresekammer. Die Proteine wurden silbergefärbt.

Um die exprimierten Antikörper immunologisch zu detektieren, wurden Western-blots auf Nitrozellulose Membranen (0.2 µm) durchgeführt. Die durch die SDS-Polyacrylamidgele aufgetrennten Proteine wurden elektro-transferiert unter Verwendung einer Novex^{®} (Gibco-BRL) Blotting-Kammer. Die Membranen wurden vor Zugabe der Block-Lösung (TBS + 3 % Trockenmilchpulver BBL) und der Antikörperlösung (10 µg/ml polyklonaler Ziegen IGN-111 Antikörper, Maus monoklonaler anti-Maus-IgG Antikörper (Zymed) oder Kaninchen anti-Maus IgG gamma Kette (Zymed) in TBS + 1% Trockenmilchpulver) zweimal gewaschen. Zum Schluß wurde die Entwicklung unter Verwendung von Kaninchen anti-Ziege-HRP, Kaninchen anti-Maus IgG-HRP oder Maus anti-Kaninchen IgG-HRP konjugiertem Antikörper (BIO-RAD) verdünnt auf 1:1000 in TBS + 1% Trockenmilchpulver durchgeführt und ein HRP Farbentwicklungsreagens (BIO-RAD) zugegeben entsprechend den Vorschriften des Herstellers.

Isoelektrische Fokusierungsgele wurden verwendet um die gereinigten Expressionsprodukte mit dem charakterisierten murinen HE2 Standard Hybridom Antikörper zu vergleichen. Die Proben wurden auf IEF Gele geladen, pH 3-7 (Invitrogen) und die Trennung gemäß den Vorschriften des Herstellers durchgeführt.

Die Proteine wurden durch Silberfärbung oder immunologische Methoden mittels Western Blot sichtbar gemacht. Für diesen Zweck wurden die Proteine einem Tris gepufferten SDS/Urea/Iodoactamide Puffer geladen und auf Nitrozellulose Membranen transferiert. Dies erfolgte nach dem gleichen Verfahren wie für Western Blots beschrieben. Der Nachweis erfolgte durch polyklonalen Ziegen IGN111 antiidiotypischen Antikörper.

Die Interaktion des Expressionsprodukts mit dem Zielantigen, EpCAM, wurde dadurch analysiert, dass die gereinigten Überstände mit Nitrozellulose Membranen auf die rEpCAM elektro-transferiert wurde, inkubiert wurden.Die Färbung der interagierenden Antikörper wurde in Analogie zu den Western Blot durchgeführt, wobei ein anti-Maus IgG2a-HRP konjugierter Antikörper (Zymed) verwendet wurde.

### Beispiel 9: Affinitätsreinigung

Ein Pharmacia (Amersham Pharmacia Biotech) ÄKTA System wurde verwendet. 1000 ml klarer Kulturüberstand enthaltend den Antikörper wurde mit einem Pro-Varion 30 kDa cut-off (Millipore) Konzentrator konzentriert, danach verdünnt mit PBS und auf eine 20 ml IGN111 Sepharose Affinitätsgel XK26/20 Säule (Amersham Pharmacia Biotech) aufgetragen. Kontaminierende Proteine wurden durch einen Waschschritt mit PBS + 200 mM NaCl entfernt. Die gebundenen Antikörper wurden mit 100 mM Glycin, pH 2.9 eluiert und sofort mit 0.5 M NaHCO₃ neutralisiert. Das Effluat wurde online bei λ 215 and λ 280 nm beobachtet und einer darauffolgenden HPLC Analyse mit einer ZORBAX G-250 (Agilent-technologies) Säule unterzogen.

2000 ml geerntete Überstände aus den Roller Bottle Kulturen wurden zentrifugiert, konzentriert, verdünnt in PBS und zur Homogenität gereinigt durch Affinitätschromatographie mit einer IGN111 Sepharose Säule. Nach Elution, Neutralisierung und Dialyse gegen PBS wurde das Endprodukt durch SEC-HPLC gemessen. Ein Hybridom-abstammender muriner Standard des selben Immunglobulins wurde mit rHE2 verglichen und eluiert, beide als scharfe einzelne peaks zur selben Zeit, korrelierend mit der erwarteten Retentionszeit von IgG. Eine Reinheit von >92 % wurde durch diese im Labormaßstab durchgeführte Reinigung erzielt.

Eine weitere Charakterisierung des Expressionsprodukts wurde durch reduzierende und nicht-reduzierende silbergefärbte SDS-PAGE und Western Blot durchgeführt. Die Expressionsprodukte wurden durch die spezifischen, antiidiotypischen Antikörper Ziege anti HE2, IGN111 detektiert und durch einen anti-Ziegen-HRP konjugierten Antikörper sichtbar gemacht. Nicht reduzierte Proben zeigten Banden in dem erwarteten Bereich eines intakten IgG Moleküls im Bereich von 160 kDa. Dieses Ergebnis korreliert exakt mit dem murinen Standard HE2 Hybridom Antikörper. Im Fall der reduzierten Proben sind Banden im Bereich von 25 bis 50 kD, auch interagierend mit dem antiidiotypischen Ziegen anti HE2 Antikörper IGN111, sichtbar. Diess Banden entsprechen den leichten und schweren Ketten des IgG.

Die Interaktion mit dem Zielantigen von HE-2, EpCAM wurde dadurch analysiert, dass Nitrozellulosemembranen, auf die rEpCAM elektro-geblottet worden ist, mit gereinigten Expressionsprodukten inkubiert wurden. Eine weitere Subtypen spezifische Detektion mit interagierenden Antikörpern wurde durchgeführt. Der murine HE-2 Standard Hybridom Antikörper erkennt monomeres rEpCAM von 25 kDa und auch eine Serie von rEpCAM Aggregaten, korrespondierend zu di, tri, and polymeren Formen. Exakt die gleiche Band Verteilung wurde mit allen gereinigten Expressionsprodukten erhalten.

Die gereinigten Expressionsprodukte und der murine HE-2 Standard Hybridom Antikörper wurden weiter untersucht. Alle Antikörper zeigen ein inhomogenes Polybanden- isoelektrisches Fokusierungsmuster, identisch in pH, aber unterschiedlich in der mengenmäßigen Verteilung. Sie bestehen aus 3 Haupt-Protein Isoformen und zwei Subformen, verteilt über einen pH Bereich von 8.2 bis 7.2. CHO abstammende Isoformen wurden auf höhere pH Werte verschoben, der murine HE2 Standard zeigt die identischen Isoformen, aber die mengenmäßige Verteilung tendiert zu sauren Formen.

Es konnte der rekombinante Maus IgG2a Antikörper HE2 in CHO Zellen exprimiert werden. Die stabile genomische Integration erfolgte 14 Tage nach Transfektion. Das Expressionskonstrukt ermöglicht eine schnelle und bequeme Transfektion mit einem einzelnen Plasmid. Durch die Verwendung des Selektionssystems basierend auf einem Host System, dem ein essentielles metabolisches Enzym fehlt, kann die Kopienzahl eines Plasmids mit dem korrespondierenden Gen und einem starken Antagonisten dieses Enzyms durch kontinuierlich ansteigenden Selektionsdruck erhöht werden. Durch die Verwendung einer attenuierten IRES Sequenz in der Expressionskasette dieses selektierbaren Markers können sehr geringe Mengen an dem Antagonisten MTX für die Selektionsstrategie verwendet werden. Moderate Expression wurde mit Mengen von 10µg /24 h.ml erreicht, welche bis mindestens 5 Wochen in den Produktionskulturen belassen warden können. Gereinigte Expressionsprodukte unterscheiden sich nicht fom murinen HE2-Standard in Größe und spezifischen immunologischen Assays. Trotzdem können Unterschiede in den post translatorischen Modifikationen auftreten. Daher zeigen rekombinante Antikörper ein Wirts- oder medienspezifisches isoelektrisches Fokusierungsmuster. Die biologische Äquivalenz des Expressionsproduktes wurde daher in weiteren Immunisierungsstudien analysiert.

### Beispiel 10: Immunisierungsstudien

### A. 17-1A Referenzgruppe

Der murine IgG2a Antikörper 17-1A (17-1A) produziert durch Hybridom Technologie wurde von Glaxo als eine 10 mg/ml PBS Lösung unter der Bezeichnung Panorex® erworben. Dieser Antikörper wurde als muriner Standard HE2 Hybridom Antikörper verwendet.

### B. rHE-2

Rekombinanter HE-2 wurde wie oben beschrieben hergestellt.

### C. Deglykosylierter 17-1A

20 mg 17-1A wurden unter nicht denaturierenden Bedingungen deglykosyliert unter Verwendung von PNGase-F (New England Biolabs, #P0704S). Die Vollständigkeit der Deglykosylierung wurde durch Western-Blot Analyse kontrolliert und durch Inkubation mit ConA-Peroxidase (Sabio#180705L1205-2). Puffertausch und Reinigung erfolgten mittels SEC Superdex 200 Chromatographie mit 1 mM NaH₂PO₄, 0.86% NaCl, pH 6.0.

### D. UPC10

UPC10, ein IgG2a Antikörper von vollkommen unterschiedlicher Spezifität wurde von Sigma (#M9144-1) gekauft.

### Formulierung der Vakzine:

Die Vakzine-Lösungen wurden in 1% Al(OH)₃ Suspensionen enthaltend 500 µg Antikörper/Dosis formuliert. Die Antikörperlösungen wurden auf Endotoxingehalt durch die LAL Endpunkt-Methode getestet. 10 and 100 µl Überstand der Lösung wurden entsprechend den Vorschriften des Herstellers getestet und verglichen mit einem Endotoxin Standard von 0.15 to 1.2 EU/ml. Antikörperlösungen wurden gegen den Formulierungspuffer 1 mM NaH₂PO₄, 0.89% NaCl, pH 6.0 dialysiert mittels einer Slide-A-Lyzer Dialysekassette 3500 MWCO, 3-15 ml (PIERCE, *#0066110).* Die Konzentration und Integrität des Proteins wurde durch SEC-HPLC (Zorbax-GF250, Agilent) getestet.

### Immunisierungsstrategie

Vier Rhesusaffen (macacca mulatta) pro Gruppe mit Körpergewichten zwischen 4 and 6 kg und ohne Vorbehandlung wurden mit 500 µl / animal s.c. am Tag 1, 15, 29 und 57 geimpft. Serumproben wurden am Tag 11, 5 und 1 (Präserum), Tag 14, Tag 29, Tag 57 und Tag 71 gesammelt.

Blutproben für die Serumpräparation wurden in Röhrchen mit Gerinnungsaktivator gesammelt und zentrifugiert 1500g für 30 Minuten (entsprechend den Anleitungen zur Verwendung). Die Serumproben wurden in Röhrchen überführt und bei -80°C aufbewahrt.

### 17-1A-ELISA

Präseren and Immunseren wurden durch ein ELISA Testsystem mit einem Immunisierungsagens für die Testung der induzierten Immunantwort analysiert. 17-1A wurde als coating Antikörper in einer Konzentration von 10 µg/ml auf Maxisorp® (NUNC) Sorptionsplatten verdünnt mit "coating buffer" (PAA, Lot: T05121-436) verwendet. Die verbleibenden aktiven Gruppen wurden durch Inkubieren mit 3% FCS (Gibco BRL, hitzeinaktiviert, #06Q6116K) in PBS blockiert, bevor die Seren in 6 x 1:10 Verdünnungen in PBS versetzt mit 2% FCS aufgetragen wurden. Die induzierten Antikörper wurden durch ihre konstanten Regionen mit einem Kaninchen-anti-humanen-IgG, A, M-HRP Konjugat (Zymed) detektiert. Die Färbung erfolgte nach üblichen Verfahren. Die Extinktion bei 492 nm wurde mit 620 nm als Referenz gemessen. Die Quantifizierung wurde durch Vergleich mit Standard Immunseren, die eine standardisierte Antikörpermenge vergleichbar mit einem Antikörpertiter von 9000 enthalten, durchgeführt.

### Affinitätstreinigung

Ein ÄKTA System (Amersham Pharmacia Biotech) wurde verwendet. 1 ml Serum wurde 1:10 mit Laufpuffer PBS versetzt mit 200 mM NaCl verdünnt und auf eine 1.0 ml 17-1A oder rEpCAM Sepharose Affinitätsgel XK10/2 Säule (Amersham Pharmacia Biotech) aufgetragen um die induzierte gesamte Immunreaktion oder das Target Antigen spezifisch zu reinigen.

Die kontaminierenden Proteine wurden durch einen Waschschritt mit PBS + 200 mM NaCl entfernt. Die gebundenen Antikörper wurden mit 100 mM Glycin, pH 2.9 eluiert und sofort mit 0.5 M NaHCO₃ neutralisiert. Das Effluat wurde online bei λ 215 und λ 280 nm gemessen. Die eluierten Fraktionen wurden anschließend einer HPLC Analyse für die Bestimmung des IgG / IgM Verhältnisses, der Reinheit und der Konzentration unterzogen.

### Resultate

Unter Berücksichtigung aller Impfungen wurden keine Nebenwirkungen beobachtet. In dieser Immunisierungsstudie führte die Vakzinierung mit verschiedenen IgG2a Formulierungen in allen Fällen zu einer starken Immunisierungsreaktion vom IgG-Typ, die Antigen spezifisch war. Mit Ausnahme der deglykosylierten 17.1A Formulierung, die zu geringerer Immunantwort führte, war die Immunogenität aller anderen Formulierungen fast die gleiche. Immuntiter stiegen von Werten unterhalb der Nachweisgrenze bis auf 300 µg/ml Serum an, was einer induzierten IgG Rate von fast 1% entspricht. Die Immunogenität aller verwendeten glykosylierten IgG2a Antikörper war fast im gleichen Bereich, unabhängig von ihrer Spezifität.

Ebenso unabhängig von der Immunisierungsgruppe bildeten alle IgG2a vakzinierten Tiere einen Immunresponse vom IgG Typ, der EpCAM erkennt mit 30-40% des immunisierungsspezifischen Antigentiters. Die Impfung mit IgG2a Antikörpern führte daher zu einer Kreuzreaktivität des Immunserums mit EpCAM. Die Deglykosylierung des immunisierenden Antigens senkte beide induzierte IgG Levels signifikant, sowohl die gerichtet gegen das immunisierende Antigen als auch die gegen EpCAM.

Die Deglykosylierung veränderte die immunogenen Eigenschaften des Antikörpers deutlich. Sowohl die Immunglobulintiter gegen das immunisierende Antigen als auch gegen das Zielantigen waren reduziert.

Der Vergleich zwischen dem ursprünglichen, vom Hybridom abstammenden Immunisierungsantigen 17-1A und dem rekombinant exprimierten rHE2 aus CHO Zellen zeigt keinerlei immunologische Unterschiede. Beide Formulierungen zeigten identische Kinetiken bei der Bildung der spezifischen Immunantwort gegen das immunisierende Antigen und das Zielantigen. Die IgG und IgM Titer, die gebildet wurden, waren ähnlich.

### Beispiel 11: Expression eines hybriden immunogenen Antikörpers

Der rekombinante IgG2a Le-Y Antikörper ist ein IgG2a Hybrid-Antikörper für die Primatenvakzinierung. Er kombiniert die antiidiotypische Lewis-Y (Le-Y) imitierende("mimicking") hypervariable Region und die hochimmunogenen Maus IgG2a konstanten Regionen.

Die rekombinante IgG2a Le-Y Antikörper Immuntherapie verstärkt die Immunogenität des ursprünglichen Antikörpers IGN301 der von einer Hybridomzelle produziert wird. Er induziert eine starke Immunantwort gegen Le-Y und / oder EpCAM überexprimiert oder präsentiert von epithelialen Tumorzellen. Diese Immunantwort führt zur Lyse der Tumorzellen durch die Komplementaktivierung oder zur Verhinderung der zellvermittelten Metastasenbildungen.

Molekularbiologische Konstrukte des rekombinanten IgG2a Le-Y Antikörpers wurden in den polycistronischen Vektor eingebaut.

Der rekombinante IgG2a Le-Y Antikörper wurde transient in HEK293 Zellen exprimiert, danach erfolgte die Calcium-Phosphat Ko-Präzipitation in einem Micro Spin System in Gegenwart von FCS. Nach der Reinigung mittels einer anti-Le-Y Affinitätssäule und Qualifizierung des Expressionsprodukts wurde der rekombinante IgG2a Le-Y Antikörper auf Al(OH)₃ formuliert and als Vakzine in Rhesusaffen Immunsierungsstudien mit vier 500 µg Dosen verwendet.

Eine hohe Immunogenität im Vergleich zur ursprünglichen IGN301 Vakzine konnte beobachtet warden. Die induzierte Immunantwort vom IgG Typ wurde durch ELISA analysiert und zeigt eine Immunisierungsantigen, Le-Y und EpCAM Spezifität.

## Patentansprüche

1. Immunogener anti-idiotypischer Antikörper, der mindestens zwei verschiedene Epitope aus Tumor-assoziierten Antigenen aufweist, wobei ein Epitop aus der Gruppe der Peptide oder Proteine und ein Epitop aus der Gruppe der Kohlenhydrate stammt, wobei der Antikörper den Idiotyp eines Antikörpers gegen ein Tumor-assoziiertes Antigen erkennt.

2. Antikörper nach Anspruch 1, **dadurch gekennzeichnet, dass** er mindestens ein Epitop eines Antigens ausgesucht aus der Gruppe der Peptide oder Proteine, insbesondere EpCAM, NCAM, CEA und T-Zell Peptide, der Kohlenhydrate, insbesondere Lewis Y, SialylTn, Globo H, und der Glycolipide, insbesondere GD2, GD3 und GM2, aufweist.

3. Antikörper nach einem der Ansprüche 1 oder 2, **dadurch gekennzeichnet, dass** er mindestens zwei Epitope von EpCAM aufweist.

4. Antikörper nach einem der Ansprüche 1 bis 3, **dadurch gekennzeichnet, dass** er mit einem Peptid, Glycopeptid, Kohlenhydrat, Lipid oder einer Nukleinsäure konjugiert ist.

5. Antikörper nach Anspruch 4, **dadurch gekennzeichnet, dass** das Peptid, Glycopeptid, Kohlenhydrat, Lipid oder die Nukleinsäure ein Epitop eines Tumor-assoziierten Antigens repräsentiert.

6. Antikörper nach einem der Ansprüche 1 bis 5, **dadurch gekennzeichnet, dass** er mindestens ein Epitop von EpCAM und mindestens ein Epitop von Lewis Y aufweist.

7. Antikörper nach einem der Ansprüche 1 bis 5, **dadurch gekennzeichnet, dass** er mindestens ein Epitop von EpCAM und mindestens ein Epitop von SialylTn aufweist.

8. Antikörper nach einem der Ansprüche 1 bis 7, **dadurch gekennzeichnet, dass** er ein humaner, humanisierter, chimärer oder muriner Antikörper ist.

9. Antikörper nach einem der Ansprüche 1 bis 8, **dadurch gekennzeichnet, dass** er ein rekombinanter Antikörper ist.

10. Antikörper nach einem der Ansprüche 1 bis 9, **dadurch gekennzeichnet, dass** er ein Antikörper-Derivat ausgesucht aus der Gruppe von Antikörper-Fragmenten, -Konjugaten, oder -Homologen ist.

11. Antikörper nach einem der Ansprüche 1 bis 10, **dadurch gekennzeichnet, dass** das Antigen des Idiotyps ausgesucht ist aus der Gruppe der Peptide oder Proteine, insbesondere EpCAM, NCAM, CEA und T-Zell Peptide.

12. Antikörper nach einem der Ansprüche 1 bis 11, **dadurch gekennzeichnet, dass** das Antigen des Idiotyps ausgesucht ist aus der Gruppe der Kohlenhydrate, insbesondere Lewis Y, SialylTn, Globo H, und der Glycolipide, insbesondere GD2, GD3 und GM2.

13. Pharmazeutische Präparation enthaltend einen immunogenen Antikörper nach einem der Ansprüche 1 bis 12.

14. Diagnostisches Mittel enthaltend einen immunogenen Antikörper nach einem der Ansprüche 1 bis 12.

15. Impfstoff-Formulierung enthaltend einen immunogenen Antikörper nach einem der Ansprüche 1 bis 12.

16. Impfstoff-Formulierung nach Anspruch 15, **dadurch gekennzeichnet, dass** der Antikörper in einer immunogenen Menge von 0.01 µg bis 10 mg enthalten ist.

17. Impfstoff-Formulierung nach einem der Ansprüche 15 oder 16, **dadurch gekennzeichnet, dass** mindestens ein Vakzine-Adjuvans enthalten ist.

18. Verfahren zur Herstellung eines immunogenen Antikörpers nach einem der Ansprüche 1 bis 12 durch
a)Bereitstellen eines Antikörpers mit dem Idiotyp eines Tumor-assoziierten Antigens und
b)Kopplung von mindestens einem Epitop eines Tumor-assoziierten Antigens oder dessen Mimik an den Antikörper.

19. Verfahren zur Herstellung eines immunogenen Antikörpers nach einem der Ansprüche 1 bis 12, durch
a)Bereitstellen einer Nukleinsäure kodierend für einen Antikörper mit dem Idiotyp eines Tumor-assoziierten Antigens und
b)Rekombinieren der Nukleinsäure mit einer Nukleinsäure, die für ein Epitop eines Tumor-assoziierten Antigens oder dessen Mimik kodiert.

20. Verfahren zur Herstellung eines immunogenen Antikörpers nach Anspruch 1, **dadurch gekennzeichnet, dass** ein Epitop eines Tumor-assoziierten Antigens oder dessen Mimik an den Antikörper als Träger konjugiert wird.

21. Verfahren nach Anspruch 20, **dadurch gekennzeichnet, dass** das Antigen ausgesucht ist aus der Gruppe der Peptide oder Proteine, insbesondere EpCAM, NCAM, CEA und T-Zell Peptide, der Kohlenhydrate, insbesondere Lewis Y, SialylTn, Globo H, und der Glycolipide, insbesondere GD2, GD3 und GM2.

22. Verfahren nach Anspruch 20 oder 21, **dadurch gekennzeichnet, dass** eine Nukleinsäure kodierend für ein Epitop eines Peptid- oder Protein-Antigens an den Antikörper konjugiert wird.

23. Verfahren nach einem der Ansprüche 20 bis 22, **dadurch gekennzeichnet, dass** der Antikörper mindestens ein weiteres Epitop eines Tumor-assoziierten Antigens aufweist.

## Claims

1. An immunogenic anti-idiotypic antibody, which comprises at least two different epitopes from tumor-associated antigens, one epitope being derived from the group of peptides or proteins and one epitope being derived from the group of carbohydrates, wherein the antibody detects the idiotype of an antibody against a tumor-associated antigen.

2. The antibody according to claim 1, **characterized in that** it comprises at least one epitope of an antigen selected from the group consisting of peptides or proteins, particularly EpCAM, NCAM, CEA and T-cell peptides, of carbohydrates, particularly Lewis Y, sialylTn, GloboH, and of glycolipids, particularly GD2, GD3 and GM2.

3. The antibody according to any one of claims 1 or 2, **characterized in that** it comprises at least two epitopes of EpCAM.

4. The antibody according to any one of claims 1 to 3, **characterized in that** it is conjugated with a peptide, glycopeptide, carbohydrate, lipid or nucleic acid.

5. The antibody according to claim 4, **characterized in that** said peptide, glycopeptide, carbohydrate, lipid or nucleic acid represents an epitope of a tumor-associated antigen.

6. The antibody according to any one of claims 1 to 5, **characterized in that** it comprises at least one epitope of EpCAM and at least one epitope of Lewis Y.

7. The antibody according to any one of claims 1 to 5, **characterized in that** it comprises at least one epitope of EpCAM and at least one epitope of sialylTn.

8. The antibody according to any one of claims 1 to 7, **characterized in that** it is a human, humanized, chimeric or murine antibody.

9. The antibody according to any one of claims 1 to 8, **characterized in that** it is a recombinant antibody.

10. The antibody according to any one of claims 1 to 9, **characterized in that** it is an antibody derivative selected from the group consisting of antibody fragments, conjugates or homologs.

11. The antibody according to any one of claims 1 to 10, **characterized in that** said antigen of the idiotype is selected from the group consisting of peptides or proteins, particularly EpCAM, NCAM, CEA and T-cell peptides.

12. The antibody according to any one of claims 1 to 11, **characterized in that** said antigen of the idiotype is selected from the group consisting of carbchydrates, particularly Lewis Y, sialylTn, GloboH, and of glycolipids, particularly GD2, GU3 and GM2.

13. A pharmaceutical preparation comprising an immunogenic antibody according to any one of claims 1 to 12.

14. A diagnostic agent comprising an immunogenic antibody according to any one of claims 1 to 12.

15. A vaccine formulation comprising an immunogenic antibody according to any one of claims 1 to 12.

16. The vaccine formulation according to claim 15, **characterized in that** said antibody is contained in an immunogenic amount of 0.01 µg to 10 mg.

17. The vaccine formulation according to any one of claims 15 or 16, **characterized in that** at least one vaccine adjuvant is contained.

18. A method for producing an immunogenic antibody according to any one of claims 1 to 12, by
a) providing an antibody including the idiotype of a tumor-associated antigen; and
b) coupling at least one epitope of a tumor-associated antigen or its mimicry to said antibody.

19. The method for producing an immunogenic antibody according to any one of claims 1 to 12, by
a) providing a nucleic acid encoding an antibody including the idiotype of a tumor-associated antigen; and
b) recombining said nucleic acid with a nucleic acid encoding an epitope of a tumor-associated antigen or its mimicry.

20. The method for producing an immunogenic antibody according to claim 1, **characterized in that** an epitope of a tumor-associated antigen or its mimicry is conjugated to said antibody as a carrier.

21. The method according to claim 20, **characterized in that** said antigen is selected from the group consisting of peptides or proteins, particularly EpCAM, NCAM, CEA and T-cell peptide, of carbohydrates, particularly Lewis Y, sialylTn, GloboH, and of glycolipids, particularly GD2, GD3 and GM2.

22. The method according to claims 20 or 21, **characterized in that** a nucleic acid encoding an epitope of a peptide or protein antigen is conjugated to said antibody.

23. The method according to any one of claims 20 to 22, **characterized in that** said antibody comprises at least one further epitope of a tumor-associated antigen.

## Revendications

1. Anticorps anti-idotypique immunogène, qui présente au moins deux épitopes différents provenant d'antigènes associés à une tumeur, un épitope provenant du groupe des peptides ou des protéines et un épitope provenant du groupe des glucides, l'anticorps reconnaissant l'idiotype d'un anticorps contre un antigène associé à une tumeur.

2. Anticorps selon la revendication 1, **caractérisé en ce qu'**il présente au moins un épitope d'un antigène choisi dans le groupe des peptides ou des protéines, en particulier des peptides EpCAM, NCAM, CEA et de lymphocytes T, des glucides, en particulier Lewis Y, SialylTn, Globo H, et des glycolipides, en particulier GD2, GD3 et GM2.

3. Anticorps selon l'une des revendications 1 ou 2, **caractérisé en ce qu'**il présente au moins deux épitopes EpCAM.

4. Anticorps selon l'une des revendications 1 à 3, **caractérisé en ce qu'**il est conjugué à un peptide, un glycopeptide, un glucide, un lipide ou un acide nucléique.

5. Anticorps selon la revendication 4, **caractérisé en ce que** le peptide, le glycopeptide, le glucide, le lipide ou l'acide nucléique représente un épitope d'un antigène associé à une tumeur.

6. Anticorps selon l'une des revendications 1 à 5, **caractérisé en ce qu'**il présente au moins un épitope d'EpCAM et au moins un épitope de Lewis Y.

7. Anticorps selon l'une des revendications 1 à 5, **caractérisé en ce qu'**il présente au moins un épitope d'EpCAM et au moins un épitope de SialylTn.

8. Anticorps selon l'une des revendications 1 à 7, **caractérisé en ce qu'**il est un anticorps humain, humanisé, chimérique ou murin.

9. Anticorps selon l'une des revendications 1 à 8, **caractérisé en ce qu'**il est un anticorps recombinant.

10. Anticorps selon l'une des revendications 1 à 9, **caractérisé en ce qu'**il est un dérivé d'anticorps choisi dans le groupe comprenant des fragments d'anticorps, des conjugués d'anticorps ou des homologues d'anticorps.

11. Anticorps selon l'une des revendications 1 à 10, **caractérisé en ce que** l'antigène de l'idiotype est choisi dans le groupe des peptides ou protéines en particulier, des peptides EpCAM, NCAM, CEA et de lymphocytes T.

12. Anticorps selon l'une des revendications 1 à 11, **caractérisé en ce que** l'antigène de l'idiotype est choisi dans le groupe des glucides, en particulier Lewis Y, SialylTn, Globo H, et des glycolipides, en particulier GD2, GD3 et GM2.

13. Préparation pharmaceutique contenant un anticorps immunogène selon l'une des revendications 1 à 12.

14. Agent diagnostique contenant un anticorps immunogène selon l'une des revendications 1 à 12.

15. Formulation vaccinale contenant un anticorps immunogène selon l'une des revendications 1 à 12.

16. Formulation vaccinale selon la revendication 15, **caractérisée en ce que** l'anticorps est contenu dans une quantité immunogène de 0,01 µg à 10 mg.

17. Formulation vaccinale selon l'une des revendications 15 ou 16, **caractérisée en ce qu'**au moins un adjuvant vaccinal est contenu.

18. Procédé de production d'un anticorps immunogène selon l'une des revendications 1 à 12 par
a) la mise à disposition d'un anticorps présentant l'idiotype d'un antigène associé à une tumeur et
b) le couplage d'au moins un épitopc d'un antigène associé à une tumeur ou son imitation à un anticorps.

19. Procédé de production d'un anticorps immunogène selon l'une des revendications 1 à 12, par
a) la mise à disposition d'un acide nucléique codant pour un anticorps présentant l'idiotype d'un antigène associé à une tumeur et
b) la recombinaison de l'acide nucléique avec un acide nucléique qui code pour un épitope d'un antigène associé à une tumeur ou son imitation.

20. Procédé de production d'un anticorps immunogène selon la revendication 1, **caractérisé en ce qu'**un épitope d'un antigène associé à une tumeur ou son imitation est conjugué à l'anticorps comme support.

21. Procédé selon la revendication 20, **caractérisé en ce que** l'antigène est choisi dans le groupe des peptides ou protéines, en particulier des peptides EpCAM, NCAM, CEA et de lymphocytes T, de glucides, en particulier Lewis Y, SialylTn, Globo H, et les glycolipides, en particulier GD2, GD3 et GM2.

22. Procédé selon la revendication 20 ou 21, **caractérisé en ce qu'**un acide nucléique codant pour un épitope d'un antigène de peptide ou de protéine est conjugué à l'anticorps.

23. Procédé selon l'une des revendications 20 à 22, **caractérisé en ce que** l'anticorps présente un autre épitope d'un antigène associé à une tumeur.
